# EUROPEAN PATENT SPECIFICATION

(11) **EP 1 315 502 B1**
(45) Date of publication and mention of the grant of the patent: **17.03.2010**
(21) Application number: 01966486.1
(22) Date of filing: 29.08.2001
(51) Int. Cl.: A61K 31/565, A61K 31/519, A61K 47/10, A61K 47/14, A61P 5/26

(54) **METHOD FOR TREATING ERECTILE DYSFUNCTION AND INCREASING LIBIDO IN MEN**
VERFAHREN ZUR BEHANDLUNG VON EREKTIONSSTÖRUNGEN UND STEIGERUNG DER LIBIDO BEI MÄNNERN
METHODE POUR TRAITER LA DYSERECTION ET AUGMENTER LA LIBIDO CHEZ L'HOMME

(30) Priority: 30.08.2000 US 651777; 01.11.2000 US 703753
(43) Date of publication of application: 04.06.2003
(73) Proprietor: Unimed Pharmaceuticals, LLC, Marietta GA 30062 (US); LABORATOIRES BESINS INTERNATIONAL, 75003 Paris (FR)
(72) Inventor: DUDLEY, Robert, E., Kenilworth, IL 60043 (US)
(74) Representative: Nargolwalla, Cyra
(86) International application number: PCT/US2001/027205
(87) International publication number: WO 2002/017927

(56) References cited:
- WO-A-00/45795
- WO-A-93/25168
- WO-A-99/66870

## Description

### RELATED APPLICATIONS

This is a continuation-in-part of U.S. Patent Application Serial No. 09/651,777, entitled, "Pharmaceutical Composition and Method for Treating Hypogonadism," filed 30 August 2000, jointly owned by Unimed Pharmaceuticals, Inc., the sole assignee of the instant application.

### BACKGROUND OF THE INVENTION

### A. Sexual Performance, Erectile Dysfunction ("ED"), and Libido in Men

### 1. Sexual Performance & ED

"Sexual performance" as used herein generally refers to a man's ability to have an orgasm, obtain an erection, or engage in masturbation or intercourse. "Impotence" is a type of deficient sexual performance. Impotence or "erectile dysfunction" as used herein is generally refers to the inability of a man to attain an erection with sufficient rigidity for vaginal penetration 25% or more of the times attempted.

As many as 45 million men have some degree of erectile dysfunction. At least 10 million American men - about 9% of the adult population - are thought to have impotence. The rate increases with age. Thus, impotence affects about 10% of men in their sixties, 25% of men in their seventies, 40% of men in their eighties, and more than half of those in their nineties. In young couples, the incidence of impotence is about 7%. One-third of older men receiving medical treatment also have difficulty with erectile function.

Over the past decade, the medical perspective on the causes of impotence has shifted. Conventional wisdom used to attribute almost all cases of impotence to psychological factors. Investigators now estimate that between 70% and 80% of impotence cases are caused primarily by medical problems. Risk factors for impotence include hypogonadism, atherosclerosis, hypertension, diabetes mellitus, depression and other emotional or psychological illnesses, pelvic surgery, kidney failure, multiple sclerosis, stroke, some types of epilepsy, and alcoholism. Another risk factor is taking any of a variety of drugs, including cardiovascular medications, drugs that affect the central nervous system, certain hormonal preparations, heroin, and cocaine.

Today, 90% of all impotence cases are treated with VIAGRA^{®} (sildenafil citrate USP). Other drugs useful in the treatment of impotence include, but are not limited to: pentoxifylline (TRENTAL^{®}), yohimbine hydrochloride (ACTIBINE^{®}, YOCON^{®}, YOHIMEX^{®}), apomorphine (UPRIMA^{®}), alprostadil (the MUSE^{®} system, TOPIGLAN^{®}, CAVERJECT^{®}), papavaerine (PAVABID^{®}, CERESPAN^{®}), and phentolamine (VASOMAX^{®}, REGITINE^{®}).

These pharmaceuticals act by a variety of physiological mechanisms. For example, the physiologic mechanism of erection of the penis involves release of nitric oxide ("NO") in the corpus cavemosum during sexual stimulation. NO then activates the enzyme guanylate cyclase, which results in increased levels of cyclic guanosine monophosphate ("cGMP"), producing smooth muscle relaxation in the corpus cavemosum and allowing inflow of blood. VIAGRA^{®} has no direct relaxant effect on isolated human corpus cavemosum, but enhances the effect of NO by inhibiting phosphodiesterase type 5 ("PDE5"), which is responsible for degradation of cGMP in the corpus cavemosum. When sexual stimulation causes local release of NO, inhibition of PDE5 by sildenafil causes increased levels of cGMP in the corpus cavemosum, resulting in smooth muscle relaxation and inflow of blood to the corpus cavemosum. In contrast, UPRIMA^{®} is a dopamine receptor agonist that acts on the central nervous system. Once absorbed and transported into the brain, UPRIMA^{®} initiates a chain of reactions that result in increased blood flow to the male genital organs and an erection. In accordance with the present invention, testosterone plays a beneficial role physiologically, and stimulates both sexual motivation (i.e., libido) and sexual performance.

### 2. Sexual Motivation and Libido

While the terms "sexual performance" and "impotence" describe physiological effects, the terms "sexual motivation" and "libido" describe psychological effects. "Libido" or "sexual motivation" as used herein is a parameter measured by the duration, frequency and extent of sexual daydreams, anticipation of sex, flirting, and sexual interaction.

As discussed above, while doctors now believe that erectile dysfunction is primarily caused by a physiological mechanism, some cases are still attributable to psychological causes. Moreover, decreased libido may also be a reaction to the experience of impotence. Unfortunately, pharmaceuticals such as VIAGRA® treat erectile dysfunction by the focusing on physiological mechanics of attaining and maintaining an erection and do little or nothing to enhance the sexual motivation or libido of men suffering from erectile dysfunction. Thus, there remains a need to treat sexual performance disorders such as impotence in a manner that overcomes both the physiological and psychological problems associated with the disorder.

An number of clinical studies involving testosterone replacement in hypogonadal males have provided convincing evidence that testosterone plays a role in both sexual motivation, libido and sexual performance. For example, researchers have reported that testosterone replacement results in increased sexual fantasies, sexual arousal and desire, spontaneous erections during sleep and in the morning, ejaculation, sexual activities with and without a partner, and orgasm through coitus or masturbation. *See generally* Christiansen, Behavioral Correlates of Testosterone, TESTOSTERONE: ACTION, DEHCIENCY, SUBSTITUTION 109-111 (1998).

### B. Testosterone Synthesis, Metabolism, and Regulation

Testosterone, the major circulating androgen in men, is synthesized from cholesterol. The approximately 500 million Leydig cells in the testes secrete more than 95% of the 6-7 mg of testosterone produced per day. Two hormones produced by the pituitary gland, luteinizing hormone ("LH") and follicle stimulating hormone ("FSH"), are required for the development and maintenance of testicular function and negatively regulate testosterone production. Circulating testosterone is metabolized to various 17-keto steroids through two different pathways. Testosterone can be metabolized to dihydrotestosterone ("DHT") by the enzyme 5α-reductase or to estradiol ("E₂") by an aromatase enzyme complex.

Testosterone circulates in the blood 98% bound to protein. In men, approximately 40% of the binding is to the high-affinity sex hormone binding globulin ("SHBC"). The remaining 60% is bound weakly to albumin. Thus, a number of measurements for testosterone are available from clinical laboratories. The term "free" testosterone as used herein refers to the fraction of testosterone in the blood that is not bound to protein. The term "total testosterone" or "testosterone" as used herein means the free testosterone plus protein-bound testosterone. The term "bioavailable testosterone" as used herein refers to the non-SHBG bound testosterone and includes that weakly bound to albumin.

The following table from the UCLA-Harbor Medical Center summarizes the hormone concentrations in normal adult men range:

**Table 1: Hormone Levels in Normal Men**

| **Hormone** | **Normal Range** |
|---|---|
| Testosterone | 298 to 1043 ng/dT |
| Free testosterone | 3.5 to 17.9 ng/dL |
| DHT | 31 to 193 ng/dL |
| DHT/T Ratio | 0.052 to 0.33 |
| DHT + T | 372 to 1349 ng/dL |
| SHBG | 10.8 to 46.6 mnol/L |
| FSH | 1.0 to 6.9 mlU/mL |
| LH | 1.0 to 8.1 mlU/mL |
| E₂ | 17.1 to 46.1 pg/mL |

There is considerable variation in the half-life of testosterone reported in the literature, ranging from 10 to 100 minutes. Researchers do agree, however, that circulating testosterone has a diurnal variation in normal young men. Maximum levels occur at approximately 6:00 to 8:00 a.m. with levels declining throughout the day. Characteristic profiles have a maximum testosterone level of 720 ng/dL and a minimum level of 430 ng/dL. The physiological significance of this diurnal cycle, if any, however, is not clear.

### C. Testosterone Levels and Sexual Behavior/Performance

Because increasing testosterone concentrations has been shown to alter sexual performance and libido, researchers have investigated methods of delivering testosterone to men. These methods include intramuscular injections (43%), oral replacement (24%), pellet implants (23%), and transdermal patches (10%). A summary of these methods is shown in Table 2.

**Table 2: Mode of Application and Dosage of Various Testosterone Preparations**

| **Preparation** | **Route Of Application** | **Full Substitution Dose** |
|---|---|---|
| **In Clinical Use** | | |
| Testosterone enanthate | Intramuscular injection | 200-25.0 g every 2-3 weeks |
| Testosterone cypionate | Intramuscular injection | 200 mg every 2 weeks |
| Testosterone undecanoate | Oral | 2-4 capsules at 40 mg per day |
| Transdermal testosterone patch | Scrotal skin | 1 membrane per day |
| Transdermal testosterone patch | Non-scrotal skin | 1 or 2 systems per day |
| Testosterone implants | Implantation under the abdominal skin | 3-6 implants of 200 mg every 6 months |

| **Under Development** | | |
|---|---|---|
| Testosterone cyclodextrin | Sublingual | 2.5-5.0 mg twice daily |
| Testosterone undecanoate | Intramuscular injection | 1000 mg every 8-10 weeks |
| Testosterone buciclate | Intramuscular injection | 1000 mg every 12-16 weeks |
| Testosterone microspheres | Intramuscular injection | 315 mg for 11 weeks |

| **Obsolete** | | |
|---|---|---|
| 17α-Methyltestosterone | Oral | 25-5.0 g per day |
| Fluoxymesterone | Sublingual | 10-25 mg per day |
| | Oral | 10-20 mg per day |

All of the testosterone replacement methods currently employed, however, suffer from one or more drawbacks. For example, subdermal pellet implants and ester injections are painful and require doctor visits. Many of these methods, such as oral/sublingual/buccal preparations, suffer from undesirable pharmacokinetic profile-creating supra-physiologic testosterone concentrations followed a return to baseline. Transdermal patches provide less than optimal pharmacokinetic characteristics, are embarrassing for many patients, and are associated with significant skin irritation. Thus, although the need for an effective testosterone replacement methodology has existed for decades, an alternative replacement therapy that overcomes these problems has never been developed.

### SUMMARY OF THE INVENTION

The present invention relates to a transdermal hydroalcoholic testosterone gel formulation for use in conjunction with pharmaceuticals aimed at treating erectile dysfunction, such as VIAGRA®, to enhance their effectiveness.

### BRIEF DESCRIPTION OF THE DRAWINGS

FIG. 1(a) is a graph showing the 24-hour testosterone pharmacokinetic profile for hypogonadal men prior to receiving 5.0 g/day of AndroGel^{®}_{,} 10.0 g/day of AndroGel^{®}, or the testosterone patch (by initial treatment group).
FIG. 1(b) is a graph showing the 24-hour testosterone pharmacokinetic profile for hypogonadal men on the first day of treatment with either 5.0 g/day of AndroGel^{®} 10.0 g/day of AndroGel^{®}, or the testosterone patch (by initial treatment group).
FIG. 1(c) is a graph showing the 24-hour testosterone pharmacokinetic profile for hypogonadal men on day 30 of treatment with either 5.0 g/day of AndroGel^{®}, 10.0 g/day of AndroGel, or the testosterone patch (by initial treatment group).
FIG. 1(d) is a graph showing the 24-hour testosterone pharmacokinetic profile for hypogonadal men on day 90 of treatment with either 5.0 g/day of AndroGel^{®}, 10.0 g/day of AndroGel^{®}, or the testosterone patch (by initial treatment group).
FIG. 1(e) is a graph showing the 24-hour testosterone pharmacokinetic profile for hypogonadal men on day 180 of treatment with either 5.0 g/day of AndroGel^{®}, 10.0 g/day of AndroGel^{®}, or the testosterone patch (by fmal treatment group).
FIG. 1(f) is a graph showing the 24-hour testosterone pharmacokinetic profile for hypogonadal men on day 0, 1, 30, 90, and 180 of treatment with 5.0 g/day of AndroGel^{®}.
FIG. 1(g) is a graph showing the 24-hour testosterone pharmacokinetic profile for hypogonadal men on day 0, 1, 30, 90, and 180 of treatment with 10.0 g/day of AndroGel^{®}.
FIG. 1(h) is a graph showing the 24-hour testosterone pharmacokinetic profile for hypogonadal men on day 0, 1, 30, 90, and 180 of treatment with the testosterone patch.
FIG. 2(a) is a graph showing the 24-hour free testosterone pharmacokinetic profile for hypogonadal men on day 1 of treatment with either 5.0 g/day of AndroGel^{®}, 10.0 g/day of AndroGel^{®}, or the testosterone patch (by initial treatment group).
FIG. 2(b) is a graph showing the 24-hour free testosterone pharmacokinetic profile for hypogonadal men on day 30 of treatment with either 5.0 g/day of AndroGel^{®}, 10.0 g/day of AndroGel^{®}, or the testosterone patch (by initial treatment group).
FIG. 2(c) is a graph showing the 24-hour free testosterone pharmacokinetic profile for hypogonadal men on day 90 of treatment with either 5.0 g/day of AndroGel^{®}, 10.0 g/day of AndroGel^{®}, or the testosterone patch (by initial treatment group).
FIG. 2(d) is a graph showing the 24-hour free testosterone pharmacokinetic profile for hypogonadal men on day 180 of treatment with either 5.0 g/day of AndroGel^{®}, 10.0 g/day of AndroGel^{®}, or the testosterone patch (by final treatment group).
FIG. 2(e) is a graph showing the 24-hour free testosterone pharmacokinetic profile for hypogonadal men on day 0, 1, 30, 90, and 180 of treatment with 5.0 g/day of AndroGel^{®}.
FIG. 2(f) is a graph showing the 24-hour free testosterone pharmacokinetic profile for hypogonadal men on day 0, 1, 30, 90, and 180 of treatment with 10.0 g/day of AndroGel^{®}.
FIG. 2(g) is a graph showing the 24-hour free testosterone pharmacokinetic profile for hypogonadal men on day 0, 1, 30, 90, and 180 of treatment with the testosterone patch.
FIG. 3 is a graph showing the DHT concentrations on days 0 through 180 for hypogonadal men receiving either 5.0 g/day of AndroGel^{®}, 10.0 g/day of AndroGel^{®}, or the testosterone patch (by initial treatment group).
FIG.4 is a graph showing the DHT/T ratio on days 0 through 180 for hypogonadal men receiving either 5.0 g/day of AndroGel^{®}, 10.0 g/day of AndroGel^{®}, or the testosterone patch (by initial treatment group).
FIG. 5 is a graph showing the total androgen concentrations (DHT +T) on days 0 through 180 for hypogonadal men receiving either 5.0 g/day of AndroGel^{®}, 10.0 g/day of AndroGel^{®}, or the testosterone patch (by initial treatment group).
FIG. 6 is a graph showing the E₂ concentrations on days 0 through 180 for hypogonadal men receiving either 5.0 g/day of AndroGel^{®}, 10.0 g/day of AndroGel^{®}, or the testosterone patch (by initial treatment group).
FIG. 7 is a graph showing the SHBG concentrations on days 0 through 180 for hypogonadal men receiving either 5.0 g/day of AndroGel^{®}, 10.0 g/day of AndroGel^{®}, or the testosterone patch (by initial treatment group).
FIG. 8(a) is a graph showing the FSH concentrations on days 0 through 180 for men having primary hypogonadism and receiving either 5.0 g/day of AndroGel^{®}, 10.0 g/day of AndroGel^{®}, or the testosterone patch (by initial treatment group).
FIG. 8(b) is a graph showing the FSH concentrations on days 0 through 180 for men having secondary hypogonadism and receiving either 5.0 g/day of AndroGel^{®}, 10.0 g/day of AndroGel^{®}, or the testosterone patch (by initial treatment group).
FIG. 8(c) is a graph showing the FSH concentrations on days 0 through 180 for men having age-associated hypogonadism and receiving either 5.0 g/day of AndroGel^{®}, 10.0 g/day of AndroGel^{®}, or the testosterone patch (by initial treatment group).
FIG. 8(d) is a graph showing the FSH concentrations on days 0 through 180 for men having hypogonadism of an unknown origin and receiving either 5.0 g/day of AndroGel^{®}, 10.0 g/day of AndroGel^{®}, or the testosterone patch (by initial treatment group).
FIG. 9(a) is a graph showing the LH concentrations on days 0 through 180 for men having primary hypogonadism and receiving either 5.0 g/day of AndroGel^{®}, 10.0 g/day of AndroGel^{®}, or the testosterone patch (by initial treatment group).
FIG. 9(b) is a graph showing the LH concentrations on days 0 through 180 for men having secondary hypogonadism and receiving either 5.0 g/day of AndroGel^{®}, 10.0 g/day of AndroGel^{®}, or the testosterone patch (by initial treatment group).
FIG. 9(c) is a graph showing the LH concentrations on days 0 through 180 for men having age-associated hypogonadism and receiving either 5.0 g/day of AndroGel^{®}, 10.0 g/day of AndroGel^{®}, or the testosterone patch (by initial treatment group).
FIG. 9(d) is a graph showing the LH concentrations on days 0 through 180 for men having hypogonadism of an unknown origin and receiving either 5.0 g/day of AndroGel^{®}, 10.0 g/day of AndroGel^{®}, or the testosterone patch (by initial treatment group).
FIG. 10(a) is a graph showing sexual motivation scores on days 0 through 180 for hypogonadal men receiving either 5.0 g/day of AndroGel^{®}, 7.5 g/day of AndroGel^{®}, 10.0 g/day of AndroGel^{®}, or the testosterone patch.
FIG. 10(b) is a graph showing overall sexual desire scores on days 0 through 180 for hypogonadal men receiving either 5.0 g/day of AndroGel^{®}, 7.5 g/day 10.0 g/day of AndroGel^{®}, or the testosterone patch.
FIG. 10(c) is a graph showing sexual enjoyment (with a partner) scores on days 0 through 180 for hypogonadal men receiving either 5.0 g/day of AndroGel^{®}, 7.5 g/day of AndroGel^{®}, 10.0 g/day of AndroGel^{®}, or the testosterone patch.
FIG. 11(a) is a graph showing sexual performance scores on days 0 through 180 for hypogonadal men receiving either 5.0 g/day of AndroGel^{®}, 7.5 g/day of AndroGel^{®}, 10.0 g/day of AndroGel^{®}, or the testosterone patch.
FIG. 11(b) is a graph showing erection satisfaction performance scores on days 0 through 180 for hypogonadal men receiving either 5.0 g/day of AndroGel^{®}, 7.5 g/day of AndroGel^{®}, 10.0 g/day of AndroGel^{®}, or the testosterone patch.
FIG. 11(c) is a graph showing percent erection scores on days 0 through 180 for hypogonadal men receiving either 5.0 g/day of AndroGel^{®}, 7.5 g/day of AndroGel^{®}, 10.0 g/day of AndroGel^{®}, or the testosterone patch.

### DETAILED DESCRIPTION OF THE INVENTION

The present invention is directed to a pharmaceutical composition for percutaneous administration comprising testosterone in a hydroalcoholic gel useful for treating erectile dysfunction and libido deficiencies. In a broad aspect of the invention, other steroids in the testosterone anabolic or catabolic pathway may be used (*e*.*g*. androstenedione, androstenediol, dehydroeplandrosterone, prenenolone, and (DHT). The gel comprises one or more lower alcohols, such as ethanol or isopropanol; a penetration enhancing agent; a thickener; and water. Additionally, the present invention may optionally include salts, emollients, stabilizers, antimicrobials, fragrances, and propellants.

A "penetration enhancer" is an agent known to accelerate the delivery of the drug through the skin. These agents also have been referred to as accelerants, adjuvants, and sorption promoters, and are collectively referred to herein as "enhancers." This class of agents includes those with diverse mechanisms of action including those which have the function of improving the solubility and diffusibility of the drug, and those which improve percutaneous absorption by changing the ability of the stratum corneum to retain moisture, softening the skin, improving the skin's permeability, acting as penetration assistants or hair-follicle openers or changing the state of the skin such as the boundary layer.

The penetration enhancer of the present invention is a functional derivative of a fatty acid, which includes isosteric modifications of fatty acids or non-acidic derivatives of the carboxylic functional group of a fatty acid or isosteric modifications thereof. In one embodiment, the functional derivative of a fatty acid is an unsaturated alkanoic acid in which the -COOH group is substituted with a functional derivative thereof, such as alcohols, polyols, amides and substituted derivatives thereof. The term "fatty acid" means a fatty acid that has four (4) to twenty-four (24) carbon atoms.

Examples of penetration enhancers include C8-C22 fatty acids such as isostearic acid, octanoic acid, and oleic acid; C8-C22 fatty alcohols such as oleyl alcohol and lauryl alcohol; lower alkyl esters of C8-C22 fatty acids such as ethyl oleate, isopropyl myristate, butyl stearate, and methyl laurate; di(lower)alkyl esters of C6-C8 diacids such as diisopropyl adipate; monoglycerides of C8-C22 fatty acids such as glyceryl monolaurate; tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; N-alkylpyrrolidone; and terpenes.

The thickeners used herein may include anionic polymers such as polyacrylic acid (CARBOPOL® by B.F. Goodrich Specialty Polymers and Chemicals Division of Cleveland, Ohio), carboxymethylcellulose and the like. Additional thickeners, enhancers and adjuvants may generally be found in Remington's The Science and Practice of Pharmacy, Meade Publishing Co., United States Pharmacopeia/National Formulary.

The composition is used in a "pharmacologically effective amount." This means that the concentration of the testosterone is such that in the composition it results in a therapeutic level of drug delivered over the term that the gel is to be used. Such delivery is dependent on a number of variables including the time period for which the individual dosage unit is to be used, the flux rate of the testosterone from the gel, surface area of application site, etc. The amount of testosterone necessary can be experimentally determined based on the flux rate of the drug through the gel, and through the skin when used with and without enhancers.

One such testosterone gel has only recently been made available in the United States under the trademark AndroGel^{®} by Unimed Pharmaceuticals, Inc., Deerfield, Illinois, the assignee of this application. In one embodiment, the gel is comprised of the following substances in approximate amounts:

**Table 3: Composition of AndroGel^{®}**

| **SUBSTANCE** | **AMOUNT (w/w) PER 100g OF GEL** |
|---|---|
| Testosterone | 1.0 g |
| Carbopol 980 | 0.90 g |
| Isopropyl myristate | 0.50 g |
| 0.1 N NaOH | 4.72 g |
| Ethanol (95% w/w) | 72.5 g* |
| Purified water (qsf) | 100 g |

| | |
|---|---|
| *Corresponding to 67 g of ethanol. | |

For example, the composition may contain 0.1 to 10.0 g of testosterone, 0.1 to 5.0 g CARBOPOL, 0.1 to 5.0 g isopropyl myristate, and 30.0 to 98.0 g ethanol.

A therapeutically effective amount of the gel is rubbed onto a given area of skin by the user. The combination of the lipophilic testosterone with the hydroalcoholic gel helps drive the testosterone into the outer layers of the skin where it is absorbed and then slowly released into the blood stream. As demonstrated by the data presented herein, the administration of the gel of the present invention has a sustained effect.

Toxicity and therapeutic efficacy of the testosterone can be determined by standard pharmaceutical procedures, *e*.*g*., for determining LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indexes are preferred. While compounds that exhibit toxic side effects may be used, care should be taken to design a delivery system that targets such compounds to the site of affected tissue in order to minimize potential damage to uninfected cells and, thereby, reduce side effects.

The present invention is further illustrated by the following examples. The practice of the present invention will employ, unless otherwise indicated, conventional techniques of pharmacology and pharmaceutics, which are within the skill of the art.

### EXAMPLES

### Reference Example 1: Improving Sexual Performance and Increasing Libido in Hypogonadal Men

In this example, hypogonadal men were recruited and studied in 16 centers in the United States. The patients were between 19 and 68 years old and had single morning serum testosterone levels at screening of less than or equal to 300 ng/dL (10.4 nmol/L ). A total of 227 patients were enrolled: 73, 78, and 76 patients were randomized to receive 5.0 g/day of AndrcGel^{®} (delivering 50 mg/day of testosterone to the skin of which about 10% or 5 mg is absorbed), 10.0 g/day of AndroGel^{®} (delivering 100 mg/day of testosterone to the skin of which about 10% or 10 mg is absorbed), or the ANDRODERM^{®} testosterone patch CT patch"; delivering 50 mg/day of testosterone), respectively.

As shown in the following table, there were no significant group-associated differences of the patients' characteristics at baseline.

**Table 4. Baseline Characteristics of the Hypogonadal Men**

| **Treatment Group** | | | | **T patch** | **AndroGel^{®} (5.0 g/day)** | **AndroGel^{®} (10.0 g/day)** |
|---|---|---|---|---|---|---|
| No of subjects enrolled | | | | 76 | 73 | 78 |
| Age (years) | | | | 51.1 | 51.3 | 51.0 |
| Range (years) | | | | 28-67 | 23-67 | 19-68 |
| Height (cm) | | | | 179.3 ± 0.9 | 175.8 ± 0.8 | 178.6 ± 0.8 |
| Weight (kg) | | | | 92.7 ± 1.6 | 90.5 ± 1.8 | 91.6 ± 1.5 |
| Serum testosterone (nmol/L) | | | | 6.40 ± 0.41 | 6.44 ± 0.39 | 6.49 ± 0.37 |
| Causes of hypogonadism | | | | | | |
| | | Primary hypogonadism | | 34 | 26 | 34 |
| | | | Klinefelter's Syndrome | 9 | 5 | 8 |
| | | | Post Orchidectomy/Anorchia | 2 | 1 | 3 |
| | | | Primary Testicular Failure | 23 | 20 | 23 |
| | | Secondary hypogonadism | | 15 | 17 | 12 |
| | | | Kallman's Syndrome | 2 | 21 | 0 |
| | | | Hypothalimic Pituitary Disorder | 6 | 6 | 3 |
| | | | Pituitary Tumor | 7 | 9 | 9 |
| | | Aging | | 6 | 13 | 6 |
| | | Not classified | | 21 | 17 | 26 |
| | Years diagnosed | | | 5.8 ± 1.1 | 4.4 ± 0.9 | 5.7 ± 1.24 |
| | Number previously treated with testosterone | | | 50 (65.8%) | 38 (52.1%) | 46 (59.0%) |
| | Type of Previous Hormonal Treatment | | | | | |
| | | Intramuscular injections | | 26 | 20 | 28 |
| | | Transdermal patch | | 12 | 7 | 8 |
| | | All others | | 12 | 11 | 10 |
| | Duration of treatment (years) | | | 5.8 ± 1.0 | 5.4 ± 0.8 | 4.6 ± 80.7 |

Forty-one percent (93/227) of the subjects had not received prior testosterone replacement therapy. Previously treated hypogonadal men were withdrawn from testosterone ester injection for at least six weeks and oral or transdermal androgens for four weeks before the screening visit. Aside from the hypogonadism, the subjects were in good health as evidenced by medical history, physical examination, complete blood count, urinalysis, and serum biochemistry. If the subjects were on lipid-lowering agents or tranquilizers, the doses were stabilized for at least three months prior to enrollment. Less than 5% of the subjects were taking supplemental calcium or vitamin D during the study. The subjects had no history of chronic medical illness, alcohol or drug abuse. They had a normal rectal examination, a PSA level of less than 4 ng/mL, and a urine flow rate of 12 mL/s or greater. Patients were excluded if they had a generalized skin disease that might affect the testosterone absorption or prior history of skin irritability with ANDRODERM^{®} patch. Subjects weighing less than 80% or over 140% of their ideal body weight were also excluded.

The randomized, multi-center, parallel study compared two doses of AndroGel^{®} with the ANDRODERM^{®} testosterone patch. The study was double-blind with respect to the AndroGel^{®} dose and open-labeled for the testosterone patch group. For the first three months of the study (days 1 to 90), the subjects were randomized to receive 5.0 g/day of AndroGel^{®}, 10.0 g/day of AndroGel^{®}, or two non-scrotal patches. In the following three months (days 91 to 180), the subjects were administered one of the following treatments: 5.0 g/day of AndroGel^{®}, 10.0 g/day of AndroGel^{®}, 7.5 g/day of AndroGel^{®}, or two non-scrotal patches. Patients who were applying AndroGel^{®} had a single, pre-application serum testosterone measured on day 60 and, if the levels were within the normal range of 300 to 1,000 ng/dL (10.4 to 34.7 nmol/L ), then they remained on their original dose. Patients with testosterone levels less than 300 ng/dL and who were originally assigned to apply 5.0 g/day of AndroGel^{®} and those with testosterone levels more than 1,000 ng/dL who had received 10.0 g/day of AndroGel^{®} were then reassigned to administer 7.5 g/day of AndroGel^{®} for days 91 to 180.

Accordingly, at 90 days, dose adjustments were made in the AndroGel^{®} groups based on the pre-application serum testosterone levels on day 60. Twenty subjects in the 5.0 g/day AndroGel^{®} group had the dose increased to 7.5 g/day. Twenty patients in the 10.0 g/day AndroGel^{®} group had the AndroGel^{®} dose reduced to 7.5 g/day. There were three patients in the testosterone patch group who were switched to 5.0 g/day AndroGel^{®} because of patch intolerance. One 10.0 g/day AndroGel^{®} subject was adjusted to receive 5.0 g/day and one 5.0 g/day AndroGel^{®} subject had the dose adjusted to 2.5 g/day. The number of subjects enrolled into day 91 to 180 of the study thus consisted of 51 receiving 5.0 g/day of AndroGel^{®}, 40 receiving 7.5 g/day of AndroGel^{®}, 52 receiving 10.0 g/day of AndroGel^{®}, and 52 continuing on the ANDRODERM^{®} patch. The treatment groups in this example may thus be characterized in two ways, either by "initial" or by the "final" treatment group. Subjects returned to the study center on days 0, 30, 60, 90, 120, 150, and 180 for a clinical examination, skin irritation and adverse event assessments.

### A. AndroGel^{®} and ANDRODERM^{®} patch

Approximately 250 g of AndroGel^{®} was packaged in multidose glass bottles that delivered 2.25 g of the gel for each actuation of the pump. Patients assigned to apply 5.0 g/day of Androgel^{®} testosterone were given one bottle of AndroGel^{®} and one bottle of placebo gel (containing vehicle but no testosterone), while those assigned to receive 10.0 g/day of AndroGel^{®} were dispensed two bottles of the active AndroGel^{®}. The patients were then instructed to apply the bottle contents to the right and left upper arms/shoulders and to the right and left sides of the abdomen on an alternate basis. For example, on the first day of the study, patients applied two actuations from one bottle, one each to the left and right upper arm/shoulder, and two actuations from the second bottle, one each to the left and right abdomen. On the following day of treatment, the applications were reversed. Alternate application sites continued throughout the study. After application of the gel to the skin, the gel dried within a few minutes. Patients washed their hands thoroughly with soap and water immediately after gel application.

The 7.5 g/day AndroGel^{®} group received their dose in an open-label fashion. After 90 days, for the subjects titrated to the AndroGel^{®} 7.5 g/day dose, the patients were supplied with three bottles, one containing placebo and the other two AndroGel^{®}. The subjects were instructed to apply one actuation from the placebo bottle and three actuations from a AndroGel^{®} bottle to four different sites of the body as above. The sites were rotated each day taking the same sequence as described above.

ANDRODERM^{®} testosterone patches each delivering 2.5 mg/day of testosterone were provided to about one-third of the patients in the study. These patients were instructed to apply two testosterone patches to a clean, dry area of skin on the back, abdomen, upper arms, or thighs once per day. Application sites were rotated with approximately seven days interval between applications to the same site.

On study days when the patients were evaluated, the gel/patches were applied following pre-dose evaluations. On the remaining days, the testosterone gel or patches were applied at approximately 8:00 a.m. for 180 days.

### B. Study Method and Results

### 1. Hormone Pharmacokinetics

On days 0, 1, 30, 90, and 180, the patients had multiple blood samples for testosterone and free testosterone measurements at 30, 15 and 0 minutes before and 2, 4, 8, 12, 16, and 24 hours after AndroGel^{®} or patch application. In addition, subjects returned on days 60, 120, and 150 for a single blood sampling prior to application of the gel or patch. Serum DHT, E₂, FSH, LH and SHBG were measured on samples collected before gel application on days 0, 30, 60, 90, 120, 150, and 180. Sera for all hormones were stored frozen at -20 °C until assay. All samples for a patient for each hormone were measured in the same assay whenever possible. The hormone assays were then measured at the Endocrine Research Laboratory of the UCLA-Harbor Medical Center.

The following table summarizes the pharmacokinetic parameters that were measured for each patient:

**Table 5: Pharmacokinetic Parameters**

| | |
|---|---|
| AUC₀₋₂₄ | area under the curve from 0 to 24 hours, determined using the linear trapezoidal rule. |
| C_{base} or Cₒ | Baseline concentration |
| C_{avg} | time-averaged concentration over the 24-hour dosing interval determined by AUC₀₋₂₄/24 |
| Cₘₐₓ | maximum concentration during the 24-hour dosing interval |
| Cₘᵢₙ | minimum concentration during the 24-hour dosing interval |
| Tₘₐₓ | time at which Cₘₐₓ occurred |
| Tₘᵢₙ | time at which Cₘᵢₙ occurred |
| Fluctuation Index | extent of variation in the serum concentration over the course of a single day, calculated as (Cₘₐₓ -Cₘᵢₙ)/C_{avg} |
| Accumulation ratio | increase in the daily drug exposure with continued dosing, calculated as the ratio of the AUC at steady state on a particular day over the AUC on day 1 (*e*.*g*., AUC_{day 30}/AUC_{day 1}) |
| Net AUC₀₋₂₄ | AUC₀₋₂₄ on days 30, 90, 180 - AUC₀₋₂₄ on day 0 |

### a. Testosterone Pharmacokinetics

### (1) Methods

Serum testosterone levels were measured after extraction with ethylacetate and hexane by a specific radioimmunoassay ("RIA") using reagents from ICN (Costa Mesa, CA). The cross reactivities of the antiserum used in the testosterone RIA were 2.0% for DHT, 2.3% for androstenedione, 0.8% for 3-β-androstanediol, 0.6% for etiocholanolone and less than 0.01 % for all other steroids tested. The lower limit of quantitation ("LLQ") for serum testosterone measured by this assay was 25 ng/dL (0.87 nmol/L). The mean accuracy of the testosterone assay, determined by spiking steroid free serum with varying amounts of testosterone (0.9 nmol/L to 52 nmol/L), was 104% and ranged from 92% to 117%. The intra-assay and inter-assay coefficients of the testosterone assay were 7.3 and 11.1%, respectively, at the normal adult male range. In normal adult men, testosterone concentrations range from 298 to 1,043 ng/dL (10.33 to 36.17 nmol/L) as determined at the UCLA-Harbor Medical Center.

### (2) Baseline Concentration

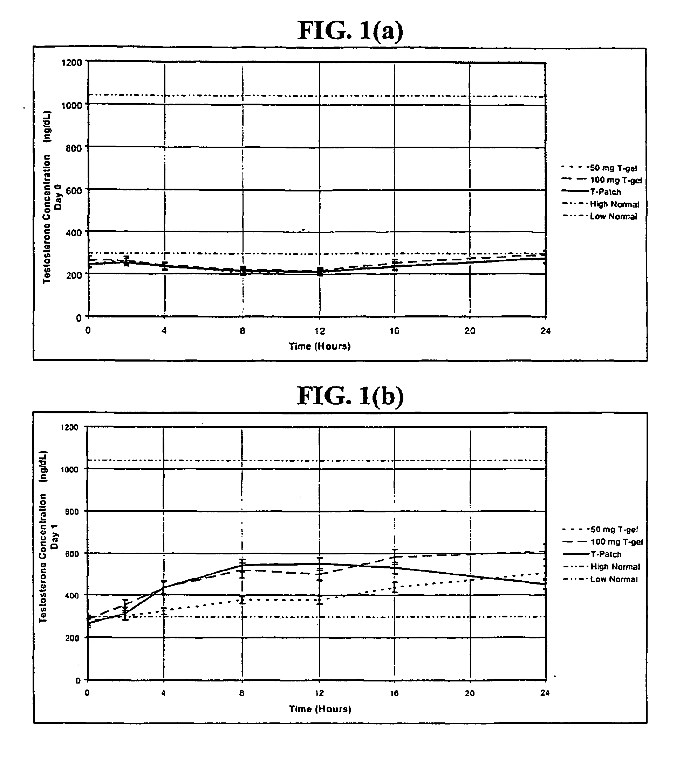

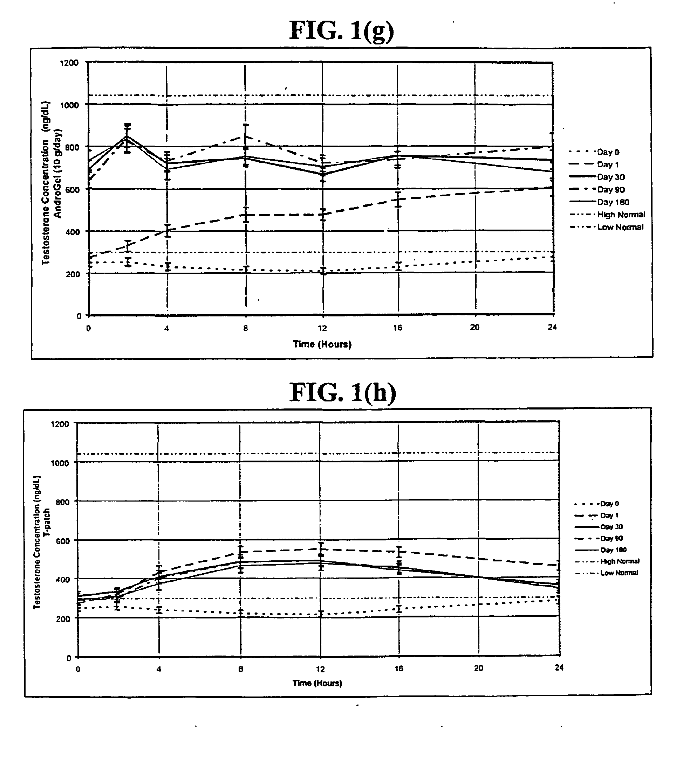

As shown in Table 6(a)-6(b) and FIG. 1(a), at baseline, the average serum testosterone concentrations over 24 hours (C_{avg}) were similar in the groups and below the adult normal range. Moreover the variations of the serum concentration (based on maximum and minimum concentrations during the 24-hour period, Cₘₐₓ and Cₘᵢₙ, respectively) during the day were also similar in the three groups. FIG. 1(a) shows that the mean testosterone levels had a the maximum level between 8 to 10 a.m. (*i*.*e*., at 0 to 2 hours) and the minimum 8 to 12 hours later, demonstrating a mild diurnal variation of serum testosterone. About one-third of the patients in each group had C_{avg} within the lower normal adult male range on day 0 (24/73 for the 5.0 g/day AndroGerl^{®} group, 25/78 for the 10.0 g/day AndroGel^{®} group, and 25/76 for testosterone patch group). All except three of the subjects met the enrollment criterion of serum testosterone less than 300 ng/dL (10.4 nmol/L) on admission.

**Table 6(a): Baseline Phamacokinetic Parameters by Initial Treatment Group (Mean ± SD)**

| | **5.0 g/day T-Gel** | **10.0 g/day T-gel** | **T-patch** |
|---|---|---|---|
| N | 73 | 78 | 76 |
| C_{avg} (ng/dL) | 237 ± 130 | 248 ± 140 | 237 ± 139 |
| Cₘₐₓ (ng/dL) | 328 ± 178 | 333 ± 194 | 314 ± 179 |
| Tₘₐₓ*(hr) | 4.0 (0.0-24.5) | 7.9 (0.0-24.7) | 4.0 (0.0-24.3) |
| Cₘᵢₙ (ng/dL) | 175 ± 104 | 188 ± 112 | 181 ± 112 |
| Tₘᵢₙ* (hr) | 8.01 (0.0-24.1) | 8.0 (0.0-24.0) | 8.0 (0.0-23.9) |
| Flue Index (ratio) | 0.627 ± 0.479 | 0.556 ± 0.384 | 0.576 ± 0.341 |

| | | | |
|---|---|---|---|
| *Median (Range*) | | | |

**Table 6(b): Baseline Testosterone Pharmacokinetic Parameters by Final Treatment Group (Mean ± SD)**

| | **Doses Received During Initial => Extended Treatment Phases** | | | | |
|---|---|---|---|---|---|
| | **5.0 g/day** | **5.0 => 7.5 g/day** | **10.0 => 7.5 g/day** | **10.0 g/day** | **T-patch** |
| | **T-gel** | **T-gel** | **T-gel** | **T-gel** | |
| N | 53 | 20 | 20 | 58 | 76 |
| C_{avg} (ng/dL) | 247 ± 137 | 212 ± 109 | 282 ± 157 | 236 ± 133 | 237 ± 140 |
| Cₘₐₓ (ng/dL) | 333 ± 180 | 313 ± 174 | 408 ± 241 | 307 ± 170 | 314 ± 179 |
| Tₘₐₓ* (hr) | 4.0 (0.0-24.5) | 4.0 (0.0-24.0) | 19.7 (0.0-24.3) | 4.0 (0.0-24.7) | 4.0 (0.0-24.3) |
| Cₘᵢₙ (ng/dL) | 185 ± 111 | 150 ± 80 | 206 ± 130 | 182 ± 106 | 181 ± 112 |
| Tₘᵢₙ* (hr) | 8.0 (0.0-24.1) | 11.9 (0.0-24.0) | 8.0 (0.0-23.3) | 8.0 (0.0-24.0) | 8.0 (0.0-23.9) |
| Fluc Index (ratio) | 0.600 ± 0.471 | 0.699 ± 0.503 | 0.678 ± 0.580 | 0.514 ± 0.284 | 0.576 ± 0.341 |

| | | | | | |
|---|---|---|---|---|---|
| *Median (range) | | | | | |

### (3) Day 1

FIG. 1(b) and Tables 6(c)-(d) show the pharmacokinetic profile for all three initial treatment groups after the first application of transdermal testosterone. In general, treatment with AndroGel^{®} and the testosterone patch produced increases in testosterone concentrations sufficiently large to bring the patients into the normal range in just a few hours. However, even on day 1, the pharmacokinetic profiles were markedly different in the AndroGel^{®} and patch groups. Serum testosterone rose most rapidly in the testosterone patch group reaching a maximum concentration (Cₘₐₓ) at about 12 hours (Tₘₐₓ). In contrast, serum testosterone rose steadily to the normal range after AndroGel^{®} application with Cₘₐₓ levels achieved by 22 and 16 hours in the 5.0 g/day AndroGel^{®} group and the 10.0 g/day AndroGel^{®} group, respectively.

**Table 6(c): Testosterone Pharmacokinetic Parameters on Day 1 by Initial Treatment Group (Mean ± SD)**

| | **5.0 g/day T-Gel** | **10.0 g/day T-gel** | **T-patch** |
|---|---|---|---|
| N | 73 | 76 | 74 |
| C_{avg} (ng/dL) | 398 ± 156 | 514 ± 227 | 482 ± 204 |
| Cₘₐₓ (ng/dL) | 560 ± 269 | 748 ± 349 | 645 ± 280 |
| Tₘₐₓ*(hr) | 22.1 (0.0-25.3) | 16.0 (0.0-24.3) | 11.8 (1.8-24.0) |
| Cₘᵢₙ (ng/dL) | 228 ± 122 | 250 ± 143 | 232 ± 132 |
| Tₘᵢₙ* (hr) | 1.9 (0.0-24.0) | 0.0 (0.0-24.2) | 1.5 (0.0-24.0) |

| | | | |
|---|---|---|---|
| *Median (Range) | | | |

**Table 6(d): Testosterone Phamacokinetic Parameters on Day 1 by Final Treatment Group (Mean ± SD)**

| | **Doses Received During Initial => Extended Treatment Phases** | | | | |
|---|---|---|---|---|---|
| | **5.0 g/day** | **5.0 => 7.5 g/day** | **10.0 => 7.5 g/day** | **10.0 g/day** | **T-patch** |
| | **T-gel** | **T-gel** | **T-gel** | **T-gel** | |
| N | 53 | 20 | 19 | 57 | 74 |
| C_{avg} (ng/dL) | 411 ± 160 | 363 ± 143 | 554 ± 243 | 500 ± 223 | 482 ± 204 |
| Cₘₐₓ (ng/dL) | 573 ± 285 | 525 ± 223 | 819 ± 359 | 724 ± 346 | 645 ± 280 |
| Tₘₐₓ* (hr) | 23.1 (0.0-25.3) | 19.5 (1.8-24.3) | 15.7 (3.9-24.0) | 23.0 (0.0-24.3) | 11.8 (1.8-24.0) |
| Cₘᵢₙ (ng/dL) | 237 ± 125 | 204 ± 112 | 265 ± 154 | 245 ± 140 | 232 ± 132 |
| Tₘᵢₙ* (hr) | 1.8 (0.0-24.0) | 3.5 (0.0-24.0) | 1.9 (0.0-24.2) | 0.0 (0.0-23.8) | 1.5 (0.0-24.0) |
| Fluc Index (ratio) | 0.600 ± 0.471 | 0.699 ± 0.503 | 0.678 ± 0.580 | 0.514 ± 0.284 | 0.576 ± 0.341 |

| | | | | | |
|---|---|---|---|---|---|
| *Median (range) | | | | | |

### (4) Days 30, 90, and 180

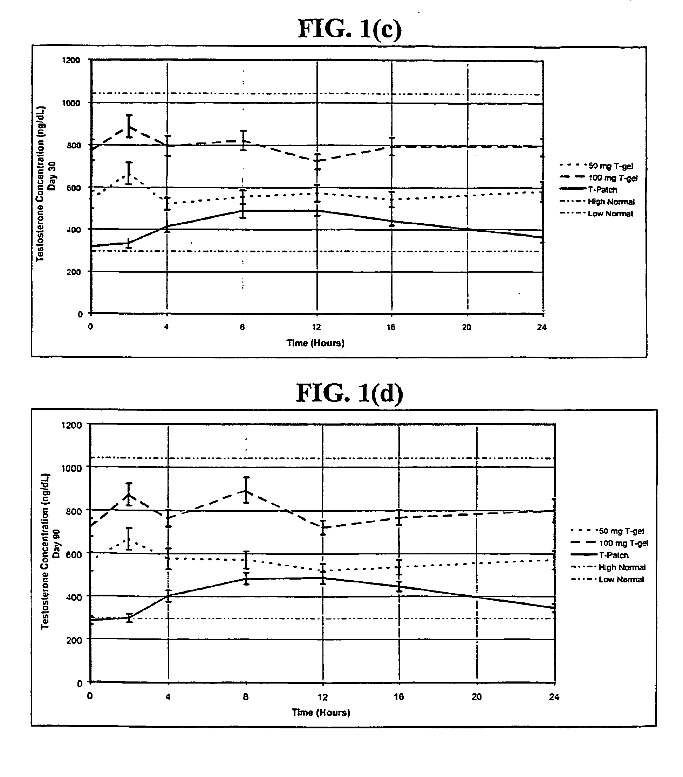

FIGS. 1(c) and 1(d) show the unique 24-hour pharmacokinetic profile of AndroGel^{®}-treated patients on days 30 and 90. In the AndroGel^{®} groups, serum testosterone levels showed small and variable increases shortly after dosing. The levels then returned to a relatively constant level. In contrast, in the testosterone patch group, patients exhibited a rise over the first 8 to 12 hours, a plateau for another 8 hours, and then a decline to the baseline of the prior day. Further, after gel application on both days 30 and 90, the C_{avg} in the 10.0 g/day AndroGel^{®} group was 1.4 fold higher than in the 5.0 g/day AndroGel^{®} group and 1.9 fold higher than the testosterone patch group. The testosterone patch group also had a Cₘᵢₙ substantially below the lower limit of the normal range. On day 30, the accumulation ratio was 0.94 for testosterone patch group, showing no accumulation. The accumulation ratios at 1.54 and 1.9 were significantly higher in the 5.0 g/day AndroGel^{®} group and 10.0 g/day AndroGel^{®} group, respectively. The differences in accumulation ratio among the groups persisted on day 90. This data indicates that the AndroGel^{®} preparations had a longer effective half-life than testosterone patch.

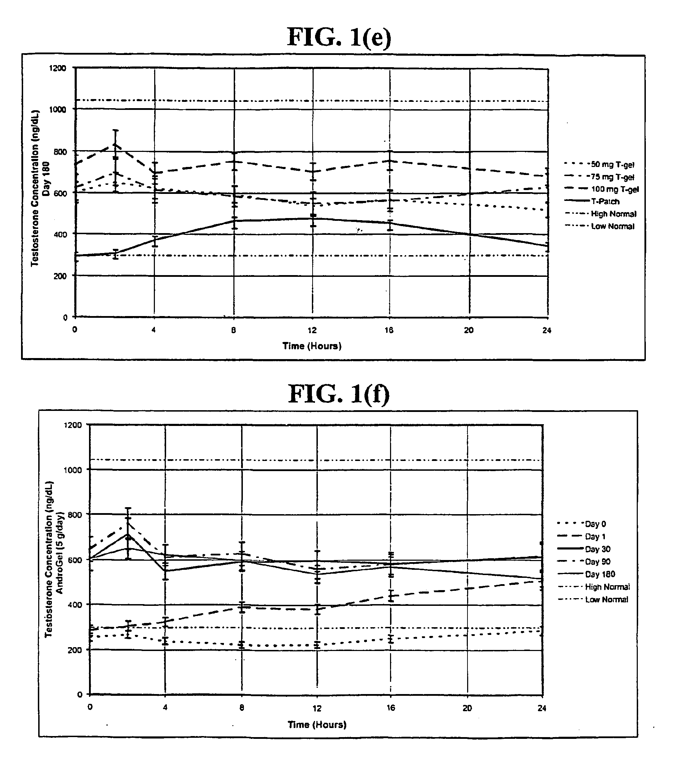

FIG. 1(e) shows the 24-hour pharmacokinetic profile for the treatment groups on day 180. In general, as Table 6(e) shows, the serum testosterone concentrations achieved and the pharmacokinetic parameters were similar to those on days 30 and 90 in those patients who continued on their initial randomized treatment groups. Table 6(f) shows that the patients titrated to the 7.5 g/day AndroGel^{®} group were not homogeneous. The patients that were previously in the 10.0 g/day group tended to have higher serum testosterone levels than those previously receiving 5.0 g/day. On day 180, the C_{avg} in the patients in the 10.0 g/day group who converted to 7.5 g/day on day 90 was 744 ng/dL, which was 1.7 fold higher than the. C_{avg} of 450 ng/dL in the patients titrated to 7.5 g/day from 5.0 g/day. Despite adjusting the dose up by 2.5 g/day in the 5.0 to 7.5 g/day group, the C_{avg} remained lower than those remaining in the 5.0 g/day group. In the 10.0 to 7.5 g/day group, the C_{avg} became similar to those achieved by patients remaining in the 10.0 g/day group without dose titration. These results suggest that many of the under-responders may actually be poorly compliant patients. For example, if a patient does not apply AndroGel^{®} properly (*e*.*g*., preferentially from the placebo container or shortly before bathing), then increasing the dose will not provide any added benefit.

FIGS. 1(f)-(h) compare the pharmacokinetic profiles for the 5.0 g/day AndroGel^{®} group, the 10.0 AndroGel^{®} g/day group, and the testosterone patch group at days 0, 1, 30, 90, and 180. In general, the mean serum testosterone levels in the testosterone patch group remained at the lower limit of the normal range throughout the treatment period. In contrast, the mean serum testosterone levels remained at about 490-570 ng/dL for the 5.0 g/day AndroGel^{®} group and about 630-860 ng/dL AndroGel^{®} for the 10.0 g/day group.

**Table 6(e): Testosterone Phamacokinetic Parameters on Day 1 by Initial Treatment Group (Mean ± SD)**

| | | **5.0 g/day T-Gel** | **10.0 g/day T-gel** | **T-patch** |
|---|---|---|---|---|
| **Day 30** | | N=66 | N=74 | N = 70 |
| | C_{avg} (ng/dL) | 566 ± 262 | 792 ± 294 | 419 ± 163 |
| | Cₘₐₓ (ng/dL) | 876 ± 466 | 1200 ± 482 | 576 ± 223 |
| | Tₘₐₓ*(hr) | 7.9 (0.0-24.0) | 7.8 (0.0-24.3) | 11.3 (0.0-24.0) |
| | Cₘᵢₙ (ng/dL) | 361 ± 149 | 505 ± 233 | 235 ± 122 |
| | Tₘᵢₙ* (hr) | 8.0 (0.0-24.1) | 8.0 (0.0-25.8) | 2.0 (0.0-24.2) |
| Fluc Index (ratio) | | 0.857 ± 0.331 | 0.895 ± 0.434 | 0.823 ± 0.289 |
| Accum Ratio (ratio) | | 1.529 ± 0.726 | 1.911 ± 1.588 | 0.937 ± 0.354 |

| **Day 90** | | N=65 | N=73 | N=64 |
|---|---|---|---|---|
| | C_{avg} (ng/dL) | 553 ± 247 | 792 ± 276 | 417 ± 157 |
| | Cₘₐₓ (ng/dL) | 846 ± 444 | 1204 ± 570 | 597 ± 242 |
| | Tₘₐₓ*(hr) | 4.0 (0.0-24.1) | 7.9 (0.0-25.2) | 8.1 (0.0-25.0) |
| | Cₘᵢₙ (ng/dL) | 354 ± 147 | 501 ± 193 | 213 ± 105 |
| | Tₘᵢₙ* (hr) | 4.0 (0.0-25.3) | 8.0 (0.0-24.8) | 2.0 (0.0-24.0) |
| Fluc Index (ratio) | | 0.851 ± 0.402 | 0.859 ± 0.399 | 0.937 ± 0.442 |
| Accum Ratio (ratio) | | 1.615 ± 0.859 | 1.927 ± 1.310 | 0.971 ± 0.453 |

| **Day 180** | | N=63 | N=68 | N=45 |
|---|---|---|---|---|
| | C_{avg} (ng/dL) | 520 ± 227 | 722 ± 242 | 403 ± 163 |
| | Cₘₐₓ (ng/dL) | 779 ± 359 | 1091 ± 437 | 580 ± 240 |
| | Tₘₐₓ*(hr) | 4.0 (0.0-24.0) | 7.9 (0.0-24.0) | 10.0 (0.0-24.0) |
| | Cₘᵢₙ (ng/dL) | 348 ± 164 | 485 ± 184 | 223 ± 114 |
| | Tₘᵢₙ* (hr) | 11.9 (0.0-24.0) | 11.8 (0.0-27.4) | 2.0 (0.0-25.7) |
| Fluc Index (ratio) | | 0.845 ± 0.379 | 0.829 ± 0.392 | 0.891 ± 0.319 |
| Accum Ratio (ratio) | | 1.523 ± 1.024 | 1.897 ± 2.123 | 0.954 ± 0.4105 |

| | | | | |
|---|---|---|---|---|
| *Median (Range) | | | | |

**Table 6(f): Testosterone Phamacokinetic Parameters on Days 30, 90, 180 by Final Treatment Group (Mean ± SD)**

| **Doses Received During Initial => Extended Treatment Phases** | | | | | |
|---|---|---|---|---|---|
| | **5.0 g/day** | **5.0 => 7.5 g/day** | **10.0 => 7.5 g/day** | **10.0 g/day** | **T-patch** |
| | **T-gel** | **T-gel** | **T-gel** | **T-gel** | |
| **Day 30** | N=47 | N= 19 | N = 19 | N = 55 | N = 70 |
| C_{avg} (ng/dL) | 604 ± 288 | 472 ± 148 | 946 ± 399 | 739 ± 230 | 419 ± 163 |
| Cₘₐₓ (ng/dL) | 941 ± 509 | 716 ± 294 | 1409 ± 556 | 1128 ± 436 | 576 ± 223 |
| Tₘₐₓ* (hr) | 7.9 (0.0-24.0) | 8.0 (0.0-24.0) | 8.0 (0.0-24.3) | 7.8 (0.0-24.3) | 11.3 (0.0-24.0) |
| Cₘᵢₙ (ng/dL) | 387 ± 159 | 296 ± 97 | 600 ± 339 | 471 ± 175 | 235 ± 122 |
| Tₘᵢₙ* (hr) | 8.1 (0.0-24.1) | 1.7 (0.0-24.1) | 11.4 (0.0-24.1) | 8.0 (0.0-25.8) | 2.0 (0.0-24.2) |
| Fluc Index (ratio) | 0.861 ± 0.341 | 0.846 ± 0.315 | 0.927 ± 0.409 | 0.884 ± 0.445 | 0.823 ± 0.289 |
| Accum Ratio (ratio) | 1.543 ± 0.747 | 1.494 ± 0.691 | 2.053 ± 1.393 | 1.864 ± 1.657 | 0.937 ± 0.354 |

| **Day 90** | N = 45 | N = 20 | N = 18 | N = 55 | N = 64 |
|---|---|---|---|---|---|
| C_{avg} (ng/dL) | 596 ± 266 | 455 ± 164 | 859 ± 298 | 771 ± 268 | 417 ± 157 |
| Cₘₐₓ (ng/dL) | 931 ± 455 | 654 ± 359 | 1398 ± 733 | 1141 ± 498 | 597 ± 242 |
| Tₘₐₓ* (hr) | 3.8 (0.0-24.1) | 7.7 (0.0-24.0) | 7.9 (0.0-24.0) | 7.9 (0.0-25.2) | 8.1 (0.0-25.0) |
| Cₘᵢₙ (ng/dL) | 384 ± 147 | 286 ± 125 | 532 ± 181 | 492 ± 197 | 213 ± 105 |
| Tₘᵢₙ* (hr) | 7.9 (0.0-25.3) | 0.0 (0.0-24.0) | 12.0 (0.0-24.1) | 4.0 (0.0-24.8) | 2.0 (0.0-24.0) |
| Fluc Index (ratio) | 0.886 ± 0.391 | 0.771 ± 0.425 | 0.959 ± 0.490 | 0.826 ± 0.363 | 0.937 ± 0.442 |
| Accum Ratio (ratio) | 1.593 ± 0.813 | 1.737 ± 1.145 | 1.752 ± 0.700 | 1.952 ± 1.380 | 0.971 ± 0.453 |

| **Day 180** | N = 44 | N = 18 | N = 19 | N = 48 | N = 41 |
|---|---|---|---|---|---|
| C_{avg} (ng/dL) | 555 ± 225 | 450 ± 219 | 744 ± 320 | 713 ± 209 | 408 ± 165 |
| Cₘₐₓ (ng/dL) | 803 ± 347 | 680 ± 369 | 1110 ± 468 | 1083 ± 434 | 578 ± 245 |
| Tₘₐₓ* (hr) | 5.8 (0.0-24.0) | 2.0 (0.0-24.0) | 7.8 (0.0-24.0) | 7.7 (0.0-24.0) | 10.6 (0.0-24.0) |
| Cₘᵢₙ (ng/dL) | 371 ± 165 | 302 ± 150 | 505 ± 233 | 485 ± 156 | 222 ± 116 |
| Tₘᵢₙ* (hr) | 11.9 (0.0-24.0) | 9.9 (0.0-24.0) | 12.0 (0.0-24.0) | 8.0 (0.0-27.4) | 2.0 (0.0-25.7) |
| Fluc Index (ratio) | 0.853 ± 0.402 | 0.833 ± 0.335 | 0.824 ± 0.298 | 0.818 ± 0.421 | 0.866 ± 0.311 |
| Accum Ratio (ratio) | 1.541 ± 0.917 | NA | NA | 2.061 ± 2.445 | 0.969 ± 0.415 |

| | | | | | |
|---|---|---|---|---|---|
| *Median (range) | | | | | |

### (5) Dose Proportionality for AndroGel^{®}

Table 6(g) shows the increase in AUC₀₋₂₄ on days 30, 90, and 180 from the pretreatment baseline (net AUC₀₋₂₄) as calculated using an arithmetic mean. In order to assess dose-proportionality, the bioequivalence assessment was performed on the log-transformed AUCs using "treatment" as the only factor. The AUCs were compared after subtracting away the AUC contribution from the endogenous secretion of testosterone (the AUC on day 0) and adjusting for the two-fold difference in applied doses. The AUC ratio on day 30 was 0.95 (90% C.I.: 0.75-1.19) and on day 90 was 0.92 (90% C.I.: 0.73-1.17). When the day 30 and day 90 data was combined, the AUC ratio was 0.93 (90% C.I.: 0.79-1.10).

The data shows dose proportionality for AndroGel^{®} treatment. The geometric mean for the increase in AUC₀₋₂₄ from day 0 to day 30 or day 90 was twice as great for the 10.0 g/day group as for the 5.0 g/day group. A 125 ng/dL mean increase in serum testosterone C_{avg} level was produced by each 2.5 g/day of AndroGel^{®}. In other words, the data shows that 0.1 g/day of AndroGel^{®} produced, on the average, a 5 ng/dL increase in serum testosterone concentration. This dose proportionality aids dosing adjustment by the physician. Because AndroGel^{®} is provided in 2.5 g packets (containing 25 mg of testosterone), each 2.5 g packet will produce, on average, a 125 ng/dL increase in the Cavg for serum total testosterone.

**Table 6(g): Net AUC₀₋₂₄ (nmol*h/L) on Days 30, 90, and 180 after Transdermal Testosterone Application**

| | **T Patch** | **T gel 5.0 g/day** | **T gel 10.0 g/day** |
|---|---|---|---|
| Day 30 | 154 ± 18 | 268 ± 28 | 446 ± 30 |
| Day 90 | 157 ± 20 | 263 ± 29 | 461 ± 28 |
| Day 180 | 160 ± 25 | 250 ± 32 | 401 ± 27 |

The increase in AUC₀₋₂₄ from pretreatment baseline achieved by the 10.0 g/day and the 5.0 g/day groups were approximately 2.7 and 1.7 fold higher than that resulting from application of the testosterone patch. These figures also indicate that an ANDRODERM^{®} patch, which produces an approximately 180 ng/dL increase in C_{avg}, is equivalent to approximately 3.5 g/day of AndroGel^{®}.

### b. Pharmacokinetics of Serum Free Testosterone Concentration

### (1) Methods

Serum free testosterone was measured by RIA of the dialysate, after an overnight equilibrium dialysis, using the same RIA reagents as the testosterone assay. The LLQ of serum free testosterone, using the equilibrium dialysis method, was estimated to be 22 pmol/L. When steroid free serum was spiked with increasing doses of testosterone in the adult male range, increasing amounts of free testosterone were recovered with a coefficient of variation that ranged from 11.0-18.5%. The intra- and interassay coefficients of free testosterone were 15% and 16.8% for adult normal male values, respectively. As estimated by the UCLA-Harbor Medical Center, free testosterone concentrations range from 3.48-17.9 ng/dL (121-620 pmol/L) in normal adult men.

### (2) Pharmacokinetic Results

In general, as shown in Table 7, the pharmacokinetic parameters of serum free testosterone mirrored that of serum total testosterone as described above. At baseline (day 0), the mean serum free testosterone concentrations (C_{avg}) were similar in all three groups which were at the lower limit of the adult male range. The maximum serum free testosterone concentration occurred between 8 and 10 a.m., and the minimum about 8 to 16 hours later. This data is consistent with the mild diurnal variation of serum testosterone.

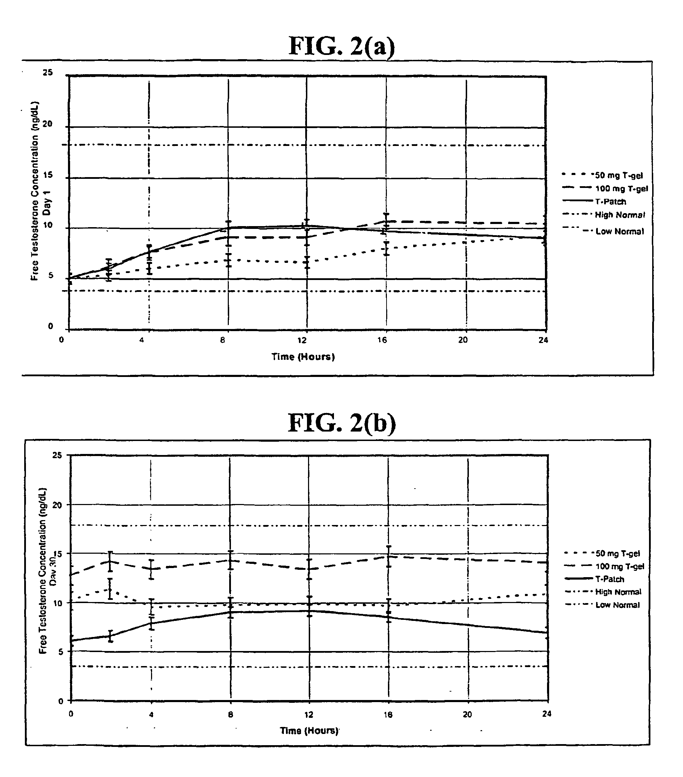

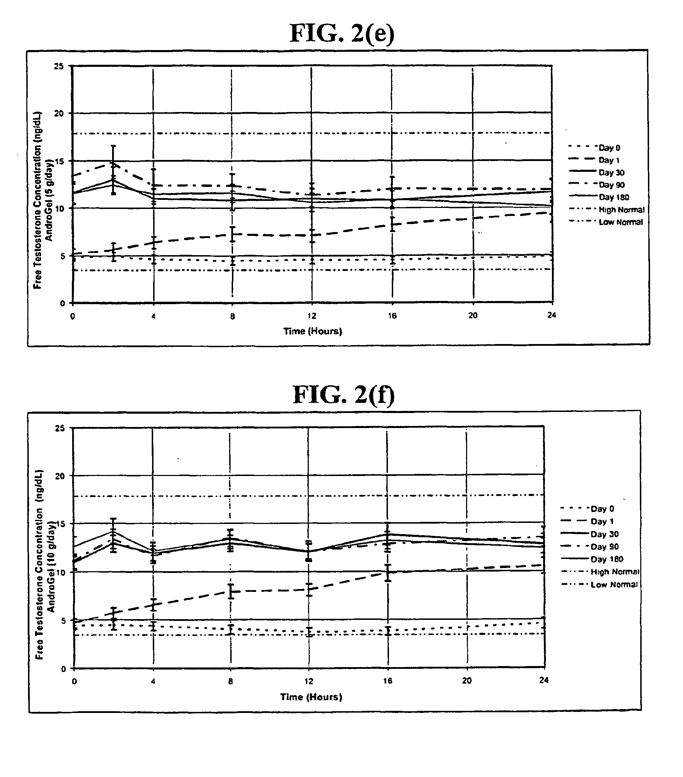

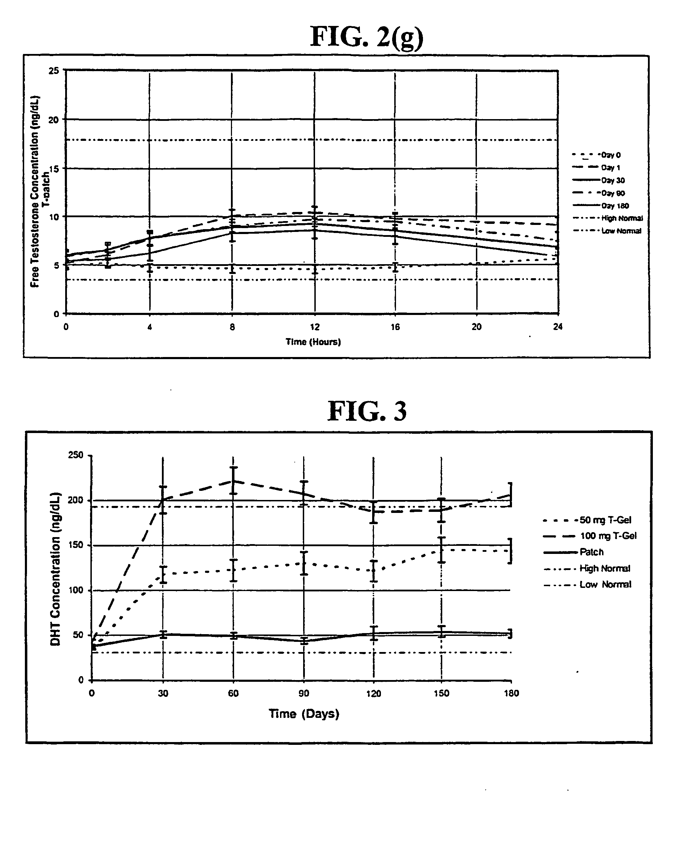

FIG. 2(a) shows the 24-hour pharmacokinetic profiles for the three treatment groups on day 1. After application of the testosterone patch, the serum free testosterone levels peaked at 12 hours about 4 hours earlier than those achieved by the AndroGel^{®} groups The serum free testosterone levels then declined in the testosterone patch group whereas in the AndroGel^{®} groups, the serum free testosterone levels continued to rise.

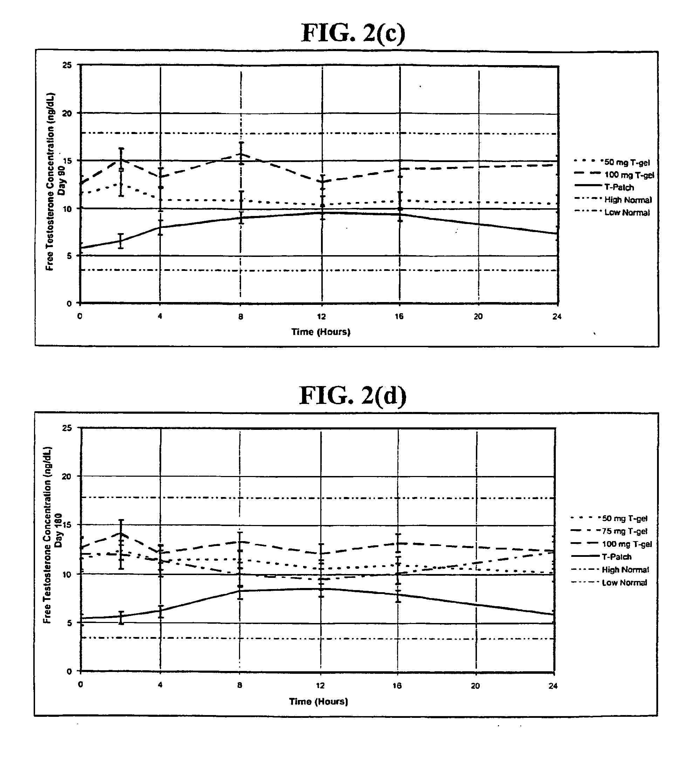

FIGS. 2(b) and 2(c) show the pharmacokinetic profiles of free testosterone in the AndroGel^{®}-treated groups resembled the unique testosterone profiles on days 30 and 90. After AndroGel^{®} application, the mean serum free testosterone levels in the three groups were within normal range. Similar to the total testosterone results, the free testosterone C_{avg} achieved by the 10.0 g/day group was 1.4 fold higher than the 5.0 g/day group and 1.7 fold higher than the testosterone patch group. Moreover, the accumulation ratio for the testosterone patch was significantly less than that of the 5.0 g/day AndroGel^{®} group and the 10.0 g/day AndroGel^{®} group.

FIG. 2(d) shows the free testosterone concentrations by final treatment groups on day 180. In general, the free testosterone concentrations exhibited a similar pattern as serum testosterone. The 24-hour pharmacokinetic parameters were similar to those on days 30 and 90 in those subjects who remained in the three original randomized groups. Again, in the subjects titrated to receive 7.5 g/day of AndroGel^{®}, the group was not homogenous. The free testosterone C_{avg} in the patients with doses adjusted upwards from 5.0 to 7.5 g/day remained 29% lower than those of subjects remaining in the 5.0 g/day group. The free testosterone C_{avg} in the patients whose doses were decreased from 10.0 to 7.5 g/day was 11% higher than those in remaining in the 10.0 g/day group.

FIGS. 2(e)-(g) show the free testosterone concentrations in the three groups of subjects throughout the 180-day treatment period. Again, the free testosterone levels followed that of total testosterone. The mean free testosterone levels in all three groups were within the normal range with the 10.0 g/day group maintaining higher free testosterone levels than both the 5.0 g/day and the testosterone patch groups.

**Table 7: Free Testosterone Pharmacokinetic Parameters by Final Treatment (Mean ± SD)**

| **Doses Received During Initial => Extended Treatment Phases** | | | | | |
|---|---|---|---|---|---|
| | **5.0 g/day** | **5.0 => 7.5 g/day** | **10.0 => 7.5 g/day** | **10/0 g/day** | **T-patch** |
| | **T-gel** | **T-gel** | **T-gel** | **T gel** | |
| **Day 0** | N = 53 | N = 20 | N = 20 | N = 58 | N = 76 |
| Cavg (ng/dL) | 4.52 ± 3.35 | 4.27 ± 3.45 | 4.64 ± 3.10 | 4.20 ± 3.33 | 4.82 ± 3.64 |
| Cmax (ng/dL) | 5.98 ± 4.25 | 6.06 ± 5.05 | 6.91 ± 4.66 | 5.84 ± 4.36 | 6.57 ± 4.90 |
| Tmax* (hr) | 4.0 (0.0-24.5) | 2.0 (0.0-24.0) | 13.5 (0.0-24.2) | 2.1 (0.0-24.1) | 3.8 (0.0-24.0) |
| Cmin (ng/dL) | 3.23 ± 2.74 | 3.10 ± 2.62 | 3.14 ± 2.14 | 3.12 ± 2.68 | 3.56 ± 2.88 |
| Tmin* (hr) | 8.0 (0.0-24.2) | 9.9 (0.0-16.0) | 4.0 (0.0-23.3) | 8.0 (0.0-24.0) | 7.9 (0.0-24.0) |
| Fluc Index (ratio) | 0.604 ± 0.342 | 0.674 ± 0.512 | 0.756 ± 0.597 | 0.634 ± 0.420 | 0.614 ± 0.362 |

| **Day 1** | N = 53 | N = 20 | N = 19 | N = 57 | N = 74 |
|---|---|---|---|---|---|
| Cavg (ng/dL) | 7.50 ± 4.83 | 6.80 ± 4.82 | 9.94 ± 5.04 | 8.93 ± 6.09 | 9.04 ± 4.81 |
| Cmax (ng/dL) | 10.86 ± 7.45 | 10.10 ± 7.79 | 15.36 ± 7.31 | 13.20 ± 8.61 | 12.02 ± 6.14 |
| Tmax* (hr) | 16.0 (0.0-25.3) | 13.9 (0.0-24.3) | 15.7 (2.0-24.0) | 23.5 (1.8-24.3) | 12.0 (1.8-24.0) |
| Cmin (ng/dL) | 4.30 ± 3.33 | 3.69 ± 3.24 | 3.88 ± 2.73 | 4.40 ± 3.94 | 4.67 ± 3.52 |
| Tmin* (hr) | 0.0 (0.0-24.1) | 1.8 (0.0-24.0) | 0.0 (0.0-24.2) | 0.0 (0.0-23.9) | 0.0 (0.0-24.0) |

| **Day 30** | N = 47 | N = 19 | N = 19 | N = 55 | N = 70 |
|---|---|---|---|---|---|
| Cavg (ng/dL) | 11.12 ± 6.22 | 7.81 ± 3.94 | 16.18 ± 8.18 | 13.37 ± 7.13 | 8.12 ± 4.15 |
| Cmax (ng/dL) | 16.93 ± 10.47 | 11.62 ± 6.34 | 25.14 ± 10.80 | 19.36 ± 9.75 | 11.48 ± 5.78 |
| Tmax* (hr) | 8.0 (0.0-27.8) | 8.0 (0.0-26.3) | 8.0 (0.0-24.3) | 8.0 (0.0-24.3) | 8.0 (0.0-24.0) |
| Cmin (ng/dL) | 6.99 ± 3.82 | 4.78 ± 3.10 | 9.99 ± 7.19 | 8.25 ± 5.22 | 4.31 ± 3.20 |
| Tmin* (hr) | 4.0 (0.0-24.1) | 3.5 (0.0-24.1) | 11.4 (0.0-24.1) | 7.8 (0.0-25.8) | 2.0 (0.0-24.8) |
| Flue Index (ratio) | 0.853 ± 0.331 | 0.872 ± 0.510 | 1.05 ± 0.449 | 0.861 ± 0.412 | 0.929 ± 0.311 |
| Accum Ratio (ratio) | 1.635 ± 0.820 | 1.479 ± 0.925 | 2.065 ± 1.523 | 1.953 ± 1.626 | 0.980 ± 0.387 |

| **Day 90** | N = 45 | N = 20 | N = 18 | N = 55 | N = 64 |
|---|---|---|---|---|---|
| Cavg (ng/dL) | 12.12 ± 7.78 | 8.06 ± 3.78 | 17.65 ± 8.62 | 13.11 ± 5.97 | 8.50 ± 5.04 |
| Cmax (ng/dL) | 18.75 ± 12.90 | 10.76 ± 4.48 | 25.29 ± 12.42 | 18.61 ± 8.20 | 12.04 ± 6.81 |
| Tmax* (hr) | 4.0 (0.0-24.0) | 9.7 (0.0-24.0) | 8.0 (0.0-24.0) | 8.0 (0.0-25.2) | 11.6 (0.0-25.0) |
| Cmin (ng/dL) | 7.65 ± 4.74 | 4.75 ± 2.86 | 10.56 ± 6.07 | 8.40 ± 4.57 | 4.38 ± 3.70 |
| Tmin* (hr) | 8.0 (0.0-24.0) | 1.9 (0.0-24.0) | 5.9 (0.0-24.1) | 4.0 (0.0-24.8) | 2.0 (0.0-14.1) |
| Fluc Index (ratio) | 0.913 ± 0.492 | 0.815 ± 0.292 | 0.870 ± 0.401 | 0.812 ± 0.335 | 0.968 ± 0.402 |
| Accum Ratio (ratio) | 1.755 ± 0.983 | 1.916 ± 1.816 | 1.843 ± 0.742 | 2.075 ± 1.866 | 1.054 ± 0.498 |

| **Day 180** | N = 44 | N = 18 | N = 19 | N = 48 | N = 41 |
|---|---|---|---|---|---|
| Cavg (ng/dL) | 11.01 ± 5.24 | 7.80 ± 4.63 | 14.14 ± 7.73 | 12.77 ± 5.70 | 7.25 ± 4.90 |
| Cmax (ng/dL) | 16.21 ± 7.32 | 11.36 ± 6.36 | 22.56 ± 12.62 | 18.58 ± 9.31 | 10.17 ± 5.90 |
| Tmax* (hr) | 7.9 (0.0-24.0) | 2.0 (0.0-23.9) | 7.8 (0.0-24.0) | 8.0 (0.0-24.0) | 11.1 (0.0-24.0) |
| Cmin (ng/dL) | 7.18 ± 3.96 | 5.32 ± 4.06 | 9.54 ± 6.45 | 8.23 ± 4.01 | 3.90 ± 4.20 |
| Tmin* (hr) | 9.9 (0.0-24.2) | 7.9 (0.0-24.0) | 8.0 (0.0-23.2) | 11.8 (0.0-27.4) | 2.5 (0.0-25.7) |
| Fluc Index (ratio) | 0.897 ± 0.502 | 0.838 ± 0.378 | 0.950 ± 0.501 | 0.81 ± 0.397 | 0.967 ± 0.370 |
| Accum Ratio (ratio) | 1.712 ± 1.071 | NA | NA | 2.134 ± 1.989 | 1.001 ± 0.580 |

| | | | | | |
|---|---|---|---|---|---|
| *Median (Range) | | | | | |

### c. Serum DHT Concentrations

Serum DHT was measured by RIA after potassium permanganate treatment of the sample followed by extraction. The methods and reagents of the DHT assay were provided by DSL (Webster, TX). The cross reactivities of the antiserum used in the RIA for DHT were 6.5% for 3-β-androstanediol, 1.2% for 3-α-androstanediol, 0.4% for 3-α-androstanediol glucuronide, and 0.4% for testosterone (after potassium permanganate treatment and extraction), and less than 0.01% for other steroids tested. This low cross-reactivity against testosterone was further confirmed by spiking steroid free serum with 35 nmol/L (1,000 pg/dL) of testosterone and taking the samples through the DHT assay. The results even on spiking with over 35 nmol/L of testosterone was measured as less than 0.1 nmol/L of DHT. The LLQ of serum DHT in the assay was 0.43 nmol/L. The mean accuracy (recovery) of the DHT assay determined by spiking steroid free serum with varying amounts of DHT from 0.43 nmol/L to 9 nmol/L was 101% and ranged from 83 to 114%. The intra-assay and inter-assay coefficients of variation for the DHT assay were 7.8 and 16.6%, respectively, for the normal adult male range. The normal adult male range of DHT was 30.7-193.2 ng/dL (1.06 to 6.66 nmol/L ) as determined by the UCLA-Harbor Medical Center.

As shown in Table 8, the pretreatment mean serum DHT concentrations were between 36 and 42 ng/dL, which were near the lower limit of the normal range in all three initial treatment groups. None of the patients had DHT concentrations above the upper limit of the normal range on the pretreatment day, although almost half (103 patients) had concentrations less than the lower limit.

FIG. 3 shows that after treatment, the differences between the mean DHT concentrations associated with the different treatment groups were statistically significant, with patients receiving AndroGel^{®} having a higher mean DHT concentration than the patients using the patch and showing dose-dependence in the mean serum DHT concentrations. Specifically, after testosterone patch application mean serum DHT levels rose to about 1.3 fold above the baseline. In contrast, serum DHT increased to 3.6 and 4.8 fold above baseline after application of 5.0 g/day and 10.0 g/day of AndroGel^{®}, respectively.

**Table 8: DHT Concentrations (ng/dL) on Each of the Observation Days By Initial Treatment (Mean ± SD)**

| | **Day 0** | **Day 30** | **Day 60** | **Day 90** | **Day 120** | **Day 150** | **Day 180** |
|---|---|---|---|---|---|---|---|
| **5.0 g/day** | N = 73 | N = 69 | N = 70 | N = 67 | N = 65 | N = 63 | N = 65 |
| **T-gel** | 36.0 ± 19.9 | 117.6 ± 74.9 | 122.4 ± 99.4 | 130.1 ± 99.2 | 121.8 ± 99.2 | 144.7 ± 110.5 | 143.7 ± 105.9 |
| **10.0 g/day** | N = 78 | N = 78 | N = 74 | N = 75 | N = 68 | N = 67 | N = 71 |
| **T-gel** | 42.0 ± 29.4 | 200.4 ± 127.8 | 222.0 ± 126.6 | 207.7 ± 111.0 | 187.3 ± 97.3 | 189.1 ± 102.4 | 206.1 ± 105.9 |
| **T-Patch** | N = 76 | N = 73 | N = 68 | N = 66 | N = 49 | N = 46 | N = 49 |
| | 37.4 ± 21.4 | 50.8 ± 34.6 | 49.3 ± 27.2 | 43.6 ± 26.9 | 53.0 ± 52.8 | 54.0 ± 42.5 | 52.1 ± 34.3 |
| Across RX | 0.6041 | 0.0001 | 0.0001 | 0.0001 | 0.0001 | 0.0001 | 0.0001 |

The increase in DHT concentrations are likely attributed to the concentration and location of 5α-reductase in the skin. For example, the large amounts of 5α-reductase in the scrotal skin presumably causes an increase in DHT concentrations in the TESTODERM^{®} patch. In contrast, the ANDRODERM^{®} and TESTODERM TTS^{®} patches create little change in DHT levels because the surface area of the patch is small and little 5α-reductase is located in nonscrotal skin. AndroGel^{®} presumably causes an increase in DHT levels because the gel is applied to a relatively large skin area and thus exposes testosterone to greater amounts of the enzyme.

To date, elevated DHT levels have not been reported to have any adverse clinical effects. Moreover, there is evidence to suggest that increased DHT levels may inhibit prostate cancer.

### d. DHT/T Ratio

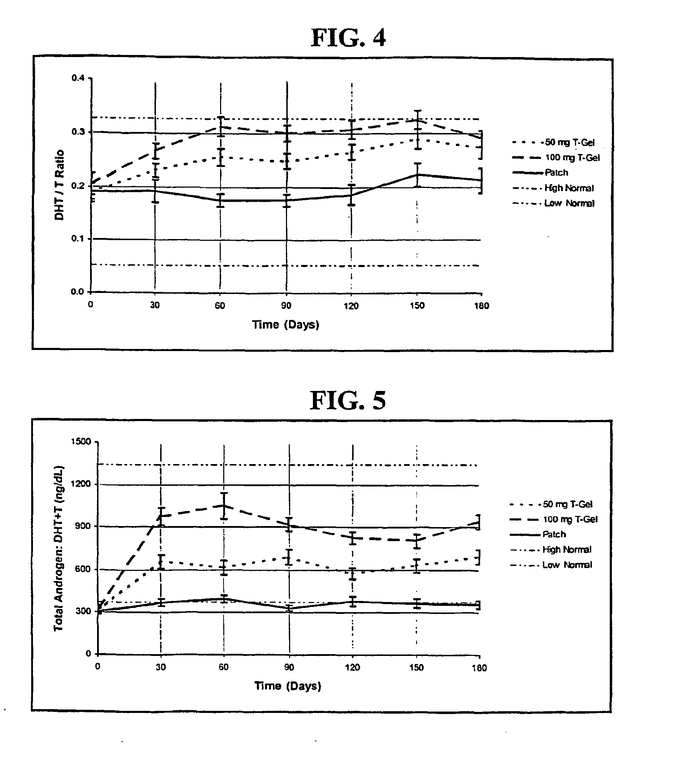

The UCLA-Harbor Medical Center reports a DHT/T ratio of 0.052-0.328 for normal adult men. In this example, the mean ratios for all three treatments were within the normal range on day 0. As shown in FIG. 4 and Table 9, there were treatment and concentration-dependent increases observed over the 180-day period. Specifically, the AndroGel^{®} treatment groups showed the largest increase in DHT/T ratio. However, the mean ratios for all of the treatment groups remained within the normal range on all observation days.

**Table 9: DHT/T Ratio on Each of the Observation Days By Initial Treatment (Mean ± SD)**

| | **Day 0** | **Day 30** | **Day 60** | **Day 90** | **Day 120** | **Day 150** | **Day 180** |
|---|---|---|---|---|---|---|---|
| **5.0 g/day** | N = 73 | N = 68 | N = 70 | N = 67 | N = 65 | N = 62 | N = 64 |
| **T-gel** | 0.198 ± 0.137 | 0.230 ± 0.104 | 0.256 ± 0.132 | 0.248 ± 0.121 | 0.266 ± 0.119 | 0.290 ± 0.145 | 0.273 ± 0.160 |
| **10.0 g/day** | N = 78 | N = 77 | N = 74 | N = 74 | N = 68 | N = 67 | N = 71 |
| **T-gel** | 0.206 ± 0.163 | 0.266 ± 0.124 | 0.313 ± 0.160 | 0.300 ± 0.131 | 0.308 ± 0.145 | 0.325 ± 0.142 | 0.291 ± 0.124 |
| **T-Patch** | N = 76 | N = 73 | N = 68 | N = 65 | N = 49 | N = 46 | N = 46 |
| | 0.204 ± 0.135 0.192 ± 0.182 | | 0.175 ± 0.102 | 0.175 ± 0.092 | 0.186 ± 0.134 | 0.223 ± 0.147 | 0.212 ± 0.160 |
| Across RX | 0.7922 | 0.0001 | 0.0001 | 0.0001 | 0.0001 | 0.0001 | 0.0002 |

### e. Total Androgen (DHT + T)

The UCLA-Harbor Medical Center has determined that the normal total androgen concentration is 372 to 1,350 ng/dL. As shown in FIG. 5 and Table 10, the mean pre-dose total androgen concentrations for all three treatments were below the lower limit of the normal range on pretreatment day 0. The total androgen concentrations for both AndroGel^{®} groups were within the normal range on all treatment observation days. In contrast, the mean concentrations for patients receiving the testosterone patch was barely within the normal range on day 60 and 120, but were below the lower normal limit on days 30, 90, 150, and 180.

**Table 10: Total Androgen (DHT +T) (ng/dL) on Each of the Observation Days By Initial Treatment (Mean ± SD)**

| | **Day 0** | **Day 30** | **Day 60** | **Day 90** | **Day 120** | **Day 150** | **Day 180** |
|---|---|---|---|---|---|---|---|
| **5.0 g/day** | N = 73 | N = 68 | N = 70 | N = 67 | N = 65 | N = 62 | N = 64 |
| **T-gel** | 281 ± 150 | 659 ± 398 | 617 ± 429 | 690 ± 431 | 574 ± 331 | 631 ± 384 | 694 ± 412 |
| **10.0 g/day** | N = 78 | N = 77 | N = 74 | N = 74 | N = 68 | N = 67 | N = 71 |
| **T-gel** | 307 ± 180 | 974 ± 532 | 1052 ± 806 | 921 ± 420 | 827 ± 361 | 805 ± 383 | 944 ± 432 |
| **T-Patch** | N = 76 | N = 73 | N = 68 | N = 65 | N = 49 | N = 46 | N = 46 |
| | 282 ± 159 | 369 ± 206 | 392 ± 229 | 330 ± 173 | 378 ± 250 | 364 ± 220 | 355 ± 202 |
| Across RX | 0.7395 | 0.0001 | 0.0001 | 0.0001 | 0.0001 | 0.0001 | 0.0001 |

### f. E₂ Concentrations

Serum E₂ levels were measured by a direct assay without extraction with reagents from ICN (Costa Mesa, CA). The intra-assay and inter-assay coefficients of variation of E₂ were 6.5 and 7.1% respectively. The UCLA-Harbor Medical Center reported an average E₂ concentration ranging from 7.1 to 46.1 pg/mL (63 to 169 pmol/L) for normal adult male range. The LLQ of the E₂ was 18 pmol/L. The cross reactivities of the E₂ antibody were 6.9% for estrone, 0.4% for equilenin, and less than 0.01% for all other steroids tested. The accuracy of the E₂ assay was assessed by spiking steroid free serum with increasing amount of E₂ (18 to 275 pmol/L). The mean recovery of E₂ compared to the amount added was 99.1 % and ranged from 95 to 101 %.

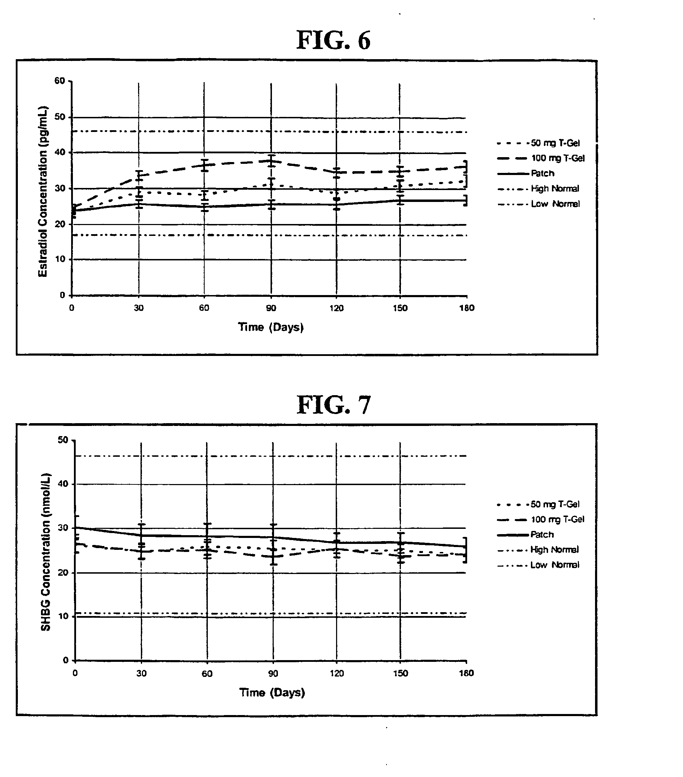

FIG. 6 depicts the E₂ concentrations throughout the 180-day study. The pretreatment mean E₂ concentrations for all three treatment groups were 23-24 pg/mL. During the study, the E₂ levels increased by an average 9.2% in the testosterone patch during the treatment period, 30.9% in the 5.0 g/day AndroGel^{®} group, and 45.5% in the 10.0 g/day AndroGel^{®} group. All of the mean concentrations fell within the normal range.

**Table 11: Estradiol Concentration (pg/mL) on Each of the Observation Days By Initial Treatment (Mean ± SD)**

| | **Day 0** | **Day 30** | **Day 60** | **Day 90** | **Day 120** | **Day 150** | **Day 180** |
|---|---|---|---|---|---|---|---|
| | N = 73 | N = 69 | N = 68 | N = 67 | N = 64 | N = 65 | N = 65 |
| **5.0 g/day T-gel** | 23.0 ± 9.2 | 29.2 ± 11.0 | 28.1 ± 10.0 | 31.4 ± 11.9 | 28.8 ± 9.9 | 30.8 ± 12.5 | 32.3 ± 13.8 |
| **10.0 g/day T-gel** | N = 78 | N = 78 | N = 74 | N = 75 | N = 71 | N = 66 | N = 71 |
| | 24.5 ± 9.5 | 33.7 ± 11.5 | 36.5 ± 13.5 | 37.8 ± 13.3 | 34.6 ± 10.4 | 35.0 ± 11.1 | 36.3 ± 13.9 |
| **T-Patch** | N = 76 | N = 72 | N = 68 | N = 66 | N = 50 | N = 49 | N = 49 |
| | 23.8 ± 8.2 | 25.8 ± 9.8 | 24.8 ± 8.0 | 25.7 ± 9.8 | 25.7±9.4 | 27.0 ± 9.2 | 26.9 ± 9.5 |
| Across RX | 0.6259 | 0.0001 | 0.0001 | 0.0001 | 0.0001 | 0.0009 | 0.0006 |

E₂ is believed to be important for the maintenance of normal bone. In addition, E₂ has a positive effect on serum lipid profiles.

### g. Serum SHBG Concentrations

Serum SHBG levels were measured with a fluoroimmunometric assay ("FIA") obtained from Delfia (Wallac, Gaithersberg, MD). The intra- and interassay coefficients were 5% and 12% respectively. The LLQ was 0.5 nmol/L. The UCLA-Harbor Medical Center determined that the adult normal male range for the SHBG assay is 0.8 to 46.6 nmol/L.

As shown in FIG. 7 and Table 12, the serum SHBG levels were similar and within the normal adult male range in the three treatment groups at baseline. None of the treatment groups showed major changes from these the baseline on any of the treatment visit days. After testosterone replacement serum SHBG levels showed a small decrease in all three groups. The most marked change occurred in the 10.0 g/day AndroGel^{®} group.

**Table 12: SHBG Concentration (nmol/L) on Each of the Observation Days By Initial Treatment (Mean ± SD)**

| | **Day 0** | **Day 30** | **Day 60** | **Day 90** | **Day 120** | **Day 150** | **Day 180** |
|---|---|---|---|---|---|---|---|
| **5.0 g/day** | N = 73 | N = 69 | N = 69 | N = 67 | N = 66 | N = 65 | N = 65 |
| **T-gel** | 26.2 ± 14.9 | 24.9 ± 14.0 | 25.9 ± 14.4 | 25.5 ± 14.7 | 25.2 ± 14.1 | 24.9 ± 12.9 | 24.2 ± 13.6 |
| **10.0 g/day** | N = 78 | N = 78 | N = 75 | N = 75 | N = 72 | N = 68 | N = 71 |
| **T-gel** | 26.6 ± 17.8 | 24.8 ± 14.5 | 25.2 ± 15.5 | 23.6 ± 14.7 | 25.5 ± 16.5 | 23.8 ± 12.5 | 24.0 ± 14.5 |
| **T-Patch** | N = 76 | N = 72 | N = 68 | N = 66 | N = 50 | N = 49 | N = 49 |
| | 30.2 ± 22.6 | 28.4 ± 21.3 | 28.2 ± 23.8 | 28.0 ± 23.6 | 26.7 ± 16.0 | 26.7 ± 16.4 | 25.8 ± 15.1 |
| Across RX | 0.3565 | 0.3434 | 0.5933 | 0.3459 | 0.8578 | 0.5280 | 0.7668 |

### h. Gonadotropins

Serum FSH and LH were measured by highly sensitive and specific solid-phase FIA assays with reagents provided by Delfia (Wallac, Gaithersburg, MD). The intra-assay coefficient of variations for LH and FSH fluroimmunometric assays were 4.3 and 5.2%, respectively; and the interassay variations for LH and FSH were 11.0% and 12.0%, respectively. For both LH and FSH assays, the LLQ was determined to be 0.2 IU/L. All samples obtained from the same subject were measured in the same assay. The UCLA-Harbor Medical Center reports that the adult normal male range for LH is 1.0-8.1 U/L and for FSH is 1.0-6.9U/L.

### (1) FSH

Table 13(a)-(d) shows the concentrations of FSH throughout the 180-day treatment depending on the cause of hypogonadism: (1) primary, (2) secondary, (3) age-associated, or (4) unknown.

Patients with primary hypogonadism show an intact feedback mechanism in that the low serum testosterone concentrations are associated with high FSH and LH concentrations. However, because of testicular or other failures, the high LH concentrations are not effective at stimulating testosterone production.

Secondary hypogonadism involves an idiopathic gonadotropin or LH-releasing hormone deficiency. Because patients with secondary hypogonadism do not demonstrate an intact feedback pathway, the lower testosterone concentrations are not associated with increased LH or FSH levels. Thus, these men have low testosterone serum levels but have gonadotropins in the normal to low range.

Hypogonadism may be age-related. Men experience a slow but continuous decline in average serum testosterone after approximately age 20 to 30 years. These untreated testosterone deficiencies in older men may lead to a variety of physiological changes. The net result is geriatric hypogonadism, or what is commonly referred to as "male menopause."

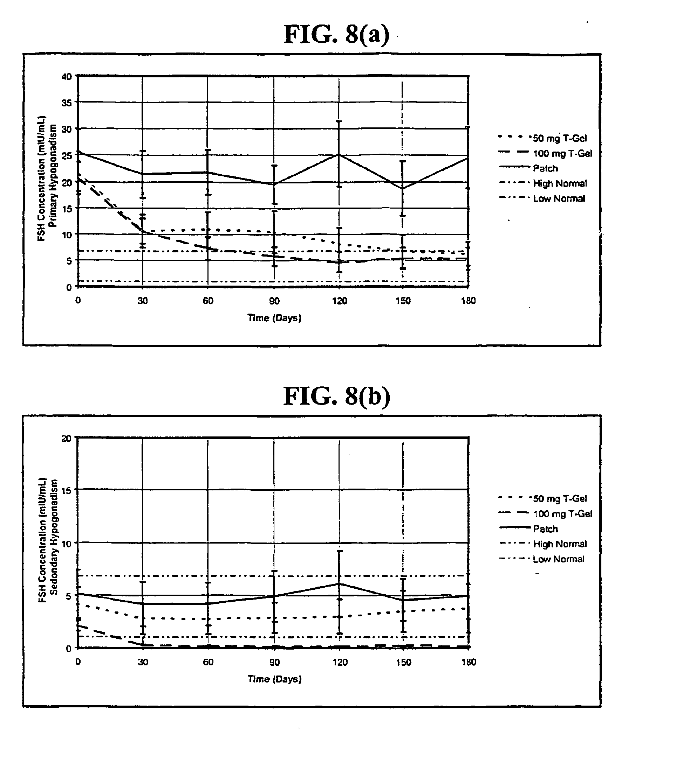

As discussed above, patients with primary hypogonadism have an intact feedback inhibition pathway, but the testes do not secrete testosterone. As a result, increasing serum testosterone levels should lead to a decrease in the serum FSH concentrations. In this example, a total of 94 patients were identified as having primary hypogonadism. For these patients, the mean FSH concentrations in the three treatment groups on day 0 were 21-26 mlU/mL, above the upper limit of the normal range. As shown in FIG. 8(a) and Table 13(a), the mean FSH concentrations decreased during treatment in all three treatment regimens. However, only the 10.0 g/day AndroGel^{®} group reduced the mean concentrations to within the normal range during the first 90 days of treatment. Treatment with the 10.0 g/day AndroGel^{®} group required approximately 120 days to reach steady state. The mean FSH concentration in patients applying 5.0 g/day of AndroGel^{®} showed an initial decline that was completed by day 30 and another declining phase at day 120 and continuing until the end of treatment. Mean FSH concentrations in the patients receiving the testosterone patch appeared to reach steady state after 30 days but were significantly higher than the normal range.

**Table 13(a): FSH Concentrations (mlU/mL) on Each of the Observation Days by Initial Treatment Group for Patients Having Primary Hypogonadism (Mean ± SD)**

| | **N** | **5 g/day** | **N** | **10 g/day** | **N** | **T-patch** |
|---|---|---|---|---|---|---|
| Day 0 | 26 | 21.6 ± 21.0 | 33 | 20.9 ± 15.9 | 34 | 25.5 ± 25.5 |
| Day 30 | 23 | 10.6 ± 15.0 | 34 | 10.6 ± 14.1 | 31 | 21.4 ± 24.6 |
| Day 60 | 24 | 10.8 ± 16.9 | 32 | 7.2 ± 12.6 | 31 | 21.7 ± 23.4 |
| Day 90 | 24 | 10.4 ± 19.7 | 31 | 5.7 ± 10.1 | 30 | 19.5 ± 20.0 |
| Day 120 | 24 | 8.1 ± 15.2 | 28 | 4.6 ± 10.2 | 21 | 25.3 ± 28.4 |
| Day 150 | 22 | 6.7 ± 15.0 | 29 | 5.3 ± 11.0 | 21 | 18.6 ± 24.0 |
| Day 180 | 24 | 6.2 ± 11.3 | 28 | 5.3 ± 11.2 | 22 | 24.5 ± 27.4 |

Patients with secondary hypogonadism have a deficient testosterone negative feedback system. As shown in FIG. 8(b), of 44 patients identified as having secondary hypogonadism, the mean FSH concentrations decreased during treatment, although the decrease over time was not statistically significant for the testosterone patch. The patients in the 5.0 g/day AndroGel^{®} group showed a decrease in the mean FSH concentration by about 35% by day 30, with no further decrease evident by day 60. Beyond day 90, the mean FSH concentration in the patients appeared to slowly return toward the pretreatment value. By day 30, all of the 10.0 g/day AndroGel^{®} group had FSH concentrations less than the lower limit.

**Table 13(b): FSH Concentrations (mlU/mL) on Each of the Observation Days by Initial Treatment Group for Patients Having Secondary Hypogonadism (Mean ± SD)**

| | **N** | **5 g/day** | **N** | **10 g/day** | **N** | **T-patch** |
|---|---|---|---|---|---|---|
| Day 0 | 17 | 4.2 ± 6.6 | 12 | 2.1 ± 1.9 | 15 | 5.1 ± 9.0 |
| Day 30 | 16 | 2.8 ± 5.9 | 12 | 0.2 ± 0.1 | 14 | 4.2 ± 8.0 |
| Day 60 | 17 | 2.8 ± 6.1 | 12 | 0.2 ± 0.1 | 13 | 4.2 ± 7.4 |
| Day 90 | 15 | 2.9 ± 5.6 | 12 | 0.2 ± 0.1 | 14 | 4.9 ± 9.0 |
| Day 120 | 14 | 3.0 ± 6.1 | 12 | 0.1 ± 0.1 | 12 | 6.1 ± 10.7 |
| Day 150 | 14 | 3.5 ± 7.5 | 12 | 0.2 ± 0.2 | 11 | 4.6 ± 6.5 |
| Day 180 | 14 | 3.7 ± 8.6 | 12 | 0.1 ± 0.1 | 12 | 4.9 ± 7.4 |

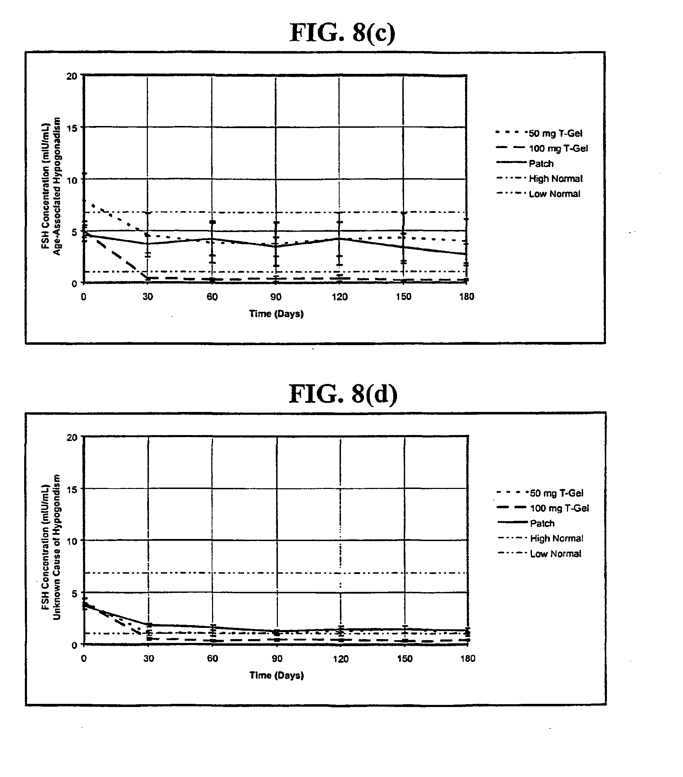

Twenty-five patients were diagnosed with age-associated hypogonadism. As shown in FIG. 8(c), the 5.0 g/day AndroGel^{®} group had a mean pretreatment FSH concentration above the normal range. The mean concentration for this group was within the normal range by day 30 and had decreased more than 50% on days 90 and 180. The decrease in FSH mean concentration in the 10.0 g/day AndroGel^{®} group showed a more rapid response. The concentrations in all six patients decreased to below the lower normal limit by day 30 and remained there for the duration of the study. The six patients who received the testosterone patch exhibited no consistent pattern in the mean FSH level; however, there was an overall trend towards lower FSH levels with continued treatment.

**Table 13(c): FSH Concentrations (mlU/mL) on Each of the Observation Days by Initial Treatment Group for Patients Having Age-Related Hypogonadism (Mean ± SD)**

| | **N** | **5 g/day** | **N** | **10 g/day** | **N** | **T-patch** |
|---|---|---|---|---|---|---|
| Day 0 | 13 | 8.0 ± 9.1 | 6 | 5.2 ± 1.9 | 6 | 4.7 ± 1.7 |
| Day 30 | 12 | 4.6 ± 7.4 | 6 | 0.4 ± 0.3 | 6 | 3.7 ± 2.0 |
| Day 60 | 12 | 3.9 ± 6.6 | 6 | 0.3 ± 0.3 | 4 | 4.3 ± 3.3 |
| Day 90 | 11 | 3.8 ± 7.0 | 6 | 0.4 ± 0.7 | 4 | 3.5 ± 1.9 |
| Day 120 | 11 | 4.2 ± 8.3 | 6 | 0.4 ± 0.7 | 4 | 4.2 ± 3.3 |
| Day 150 | 11 | 4.3 ± 8.1 | 5 | 0.2 ± 0.2 | 4 | 3.4 ± 2.7 |
| Day 180 | 11 | 4.0 ± 7.2 | 6 | 0.2 ± 0.2 | 4 | 2.7 ± 2.1 |

Sixty-four patients in the study suffered from unclassified hypogonadism. As shown in FIG. 8(d), the patients showed a marked and comparatively rapid FSH concentration decrease in all three groups, with the greatest decrease being in the 10.0 g/day AndroGel^{®} group. The 10.0 g/day AndroGel^{®} group produced nearly a 90% decrease in the mean FSH concentration by day 30 and maintained the effect to day 180. The 5.0 g/day AndroGel^{®} group produced about a 75% drop in mean FSH concentration by day 30 and stayed at that level for the remainder of treatment. The 21 patients receiving the testosterone patch had a 50% decrease in the mean FSH concentration by day 30, a trend that continued to day 90 when the concentration was about one-third of its pretreatment value.

**Table 13(d): Concentrations (mlU/mL) for FSH on Each of the Observation Days by Initial Treatment Group for Patients Having Unknown-Related Hypogonadism (Mean ± SD)**

| | **N** | **5 g/day** | **N** | **10 g/day** | **N** | **T-patch** |
|---|---|---|---|---|---|---|
| Day 0 | 17 | 4.0 ± 1 .8 | 26 | 4.1 ± 1.6 | 21 | 3.7 ± 1.4 |
| Day 30 | 17 | 1.1 ± 1.0 | 26 | 0.5 ± 0.5 | 21 | 1.8 ± 0.8 |
| Day 60 | 16 | 1.1 ± 1.1 | 26 | 0.3 ± 0.3 | 18 | 1.6 ± 1.0 |
| Day 90 | 17 | 1.1 ± 1.1 | 25 | 0.4 ± 0.7 | 18 | 1.2 ± 0.9 |
| Day 120 | 16 | 1.2 ± 1.4 | 26 | 0.4 ± 0.6 | 12 | 1.4 ± 1.0 |
| Day 150 | 17 | 1.4 ± 1.4 | 23 | 0.3 ± 0.5 | 13 | 1.4 ± 1.2 |
| Day 180 | 16 | 1.0 ± 0.9 | 24 | 0.4 ± 0.4 | 11 | 1.3 ± 0.9 |

This data shows that feedback inhibition of FSH secretion functioned to some extent in all four subpopulations. The primary hypogonadal population showed a dose-dependency in both the extent and rate of the decline in FSH levels. The sensitivity of the feedback process appeared to be reduced in the secondary and age-associated groups in that only the highest testosterone doses had a significant and prolonged impact on FSH secretion. In contrast, the feedback inhibition pathway in the patients in the unclassified group was quite responsive at even the lowest dose of exogenous testosterone.

### (2) LH

The response of LH to testosterone was also examined separately for the same four subpopulations. Table 14(a)-(d) shows the LH concentrations throughout the treatment period.

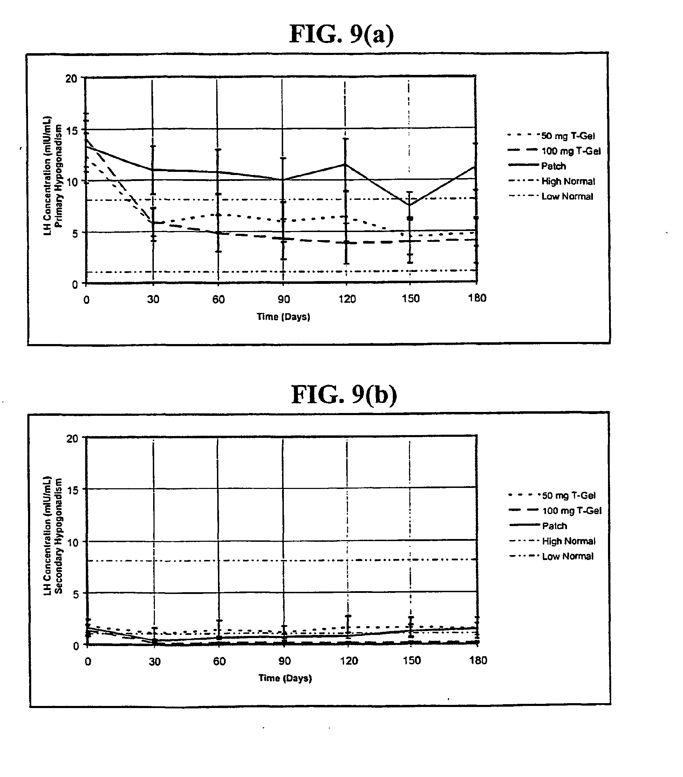

As shown in FIG. 9(a) and Table 14(a), the LH concentrations prior to treatment were about 175% of the upper limit of the normal range in primary hypogonadal patients. The mean LH concentrations decreased during treatment in all groups. However, only the AndroGel^{®} groups decreased the mean LH concentrations enough to fall within the normal range. As with FSH, the primary hypogonadal men receiving AndroGel^{®} showed dose-dependence in both the rate and extent of the LH response.

**Table 14(a): Concentrations for LH (mlU/mL) on Each of the Observation Days for Patients Having Primary Hypogonadism (Summary of Mean ± SD)**

| | **N** | **5 g/day** | **N** | **10 g/day** | **N** | **T-patch** |
|---|---|---|---|---|---|---|
| Day 0 | 26 | 12.2 ± 12.1 | 33 | 13.9 ± 14.9 | 33 | 13.3 ± 14.3 |
| Day 30 | 23 | 5.6 ± 7.6 | 34 | 5.9 ± 8.1 | 31 | 10.9 ± 12.9 |
| Day 60 | 24 | 6.8 ± 9.0 | 32 | 4.8 ± 10.0 | 31 | 10.8 ± 11.8 |
| Day 90 | 24 | 5.9 ± 9.5 | 31 | 4.2 ± 11.0 | 30 | 10.0 ± 11.7 |
| Day 120 | 24 | 6.4 ± 11.9 | 28 | 3.8 ± 10.4 | 21 | 11.5 ± 11.5 |
| Day 150 | 22 | 4.4 ± 8.5 | 29 | 4.0 ± 11.3 | 21 | 7.4 ± 6.0 |
| Day 180 | 24 | 4.8 ± 6.8 | 28 | 4.0 ± 11.9 | 22 | 11.2 ± 10.5 |

The secondary hypogonadal men were less sensitive to exogenous testosterone. For the 44 patients identified as having secondary hypogonadism, the pretreatment mean concentrations were all within the lower limit normal range. The mean LH concentrations decreased during treatment with all three regimens as shown in FIG. 9(b) and Table 14(b).

**Table 14(b): Concentrations for LH (mlU/mL) on Each of the Observation Days for Patients Having Secondary Hypogonadism (Summary of Mean ± SD)**

| | **N** | **5 g/day** | **N** | **10 g/day** | **N** | **T-patch** |
|---|---|---|---|---|---|---|
| Day 0 | 17 | 1.8 ± 2.6 | 12 | 1.4 ± 1.8 | 15 | 1.6 ± 3.1 |
| Day 30 | 16 | 1.1 ± 2.2 | 12 | 0.2 ± 0.2 | 14 | 0.4 ± 0.4 |
| Day 60 | 17 | 1.4 ± 3.8 | 12 | 0.2 ± 0.2 | 13 | 0.6 ± 0.5 |
| Day 90 | 15 | 1.2 ± 2.4 | 12 | 0.2 ± 0.2 | 14 | 0.7 ± 1.0 |
| Day 120 | 14 | 1.6 ± 4.0 | 12 | 0.2 ± 0.2 | 12 | 0.8 ± 0.8 |
| Day 150 | 14 | 1.6 ± 3.5 | 12 | 0.2 ± 0.2 | 11 | 1.2 ± 2.0 |
| Day 180 | 14 | 1.5 ± 3.7 | 12 | 0.2 ± 0.2 | 12 | 1.4 ± 2.1 |

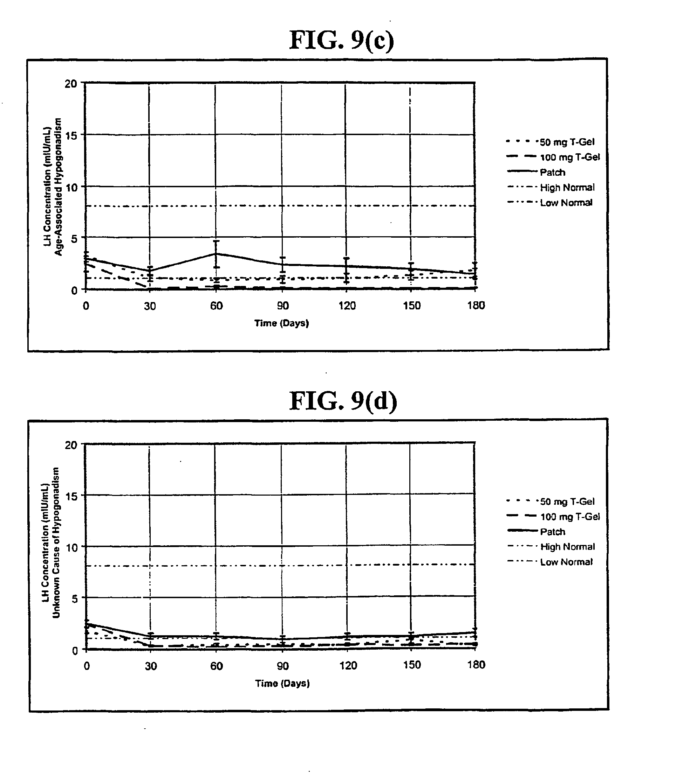

None of the 25 patients suffering from age-associated hypogonadism had pretreatment LH concentrations outside of the normal range as shown in FIG. 9(c) and Table 14(c). The overall time and treatment effects were significant for the AndroGel^{®} patients but not those patients using the testosterone patch.

**Table 14(c): Concentrations for LH (mlU/mL) on Each of the Observation Days for Patients Having Age-Related Hypogonadism (Summary of Mean ± SD)**

| | **N** | **5 g/day** | **N** | **10 g/day** | **N** | **T-patch** |
|---|---|---|---|---|---|---|
| Day 0 | 13 | 3.2 ± 1.1 | 6 | 2.4 ± 1.8 | 6 | 2.9 ± 0.6 |
| Day 30 | 12 | 1.1 ± 1.0 | 6 | 0.1 ± 0.0 | 6 | 1.8 ± 1.1 |
| Day 60 | 12 | 0.8 ± 0.7 | 6 | 0.2 ± 0.3 | 5 | 3.4 ± 2.8 |
| Day 90 | 11 | 0.9 ± 1.2 | 6 | 0.1 ± 0.0 | 4 | 2.3 ± 1.4 |
| Day 120 | 11 | 1.0 ± 1.4 | 6 | 0.1 ± 0.0 | 4 | 2.2 ± 1.4 |
| Day 150 | 11 | 1.3 ± 1.5 | 5 | 0.1 ± 0.0 | 4 | 1.9 ± 1.2 |
| Day 180 | 11 | 1.8 ± 2.1 | 6 | 0.1 ± 0.0 | 4 | 1.4 ± 1.0 |

Of the 64 patients suffering from an unclassified hypogonadism, none of the patients had a pretreatment LH concentration above the upper limit. Fifteen percent, however, had pretreatment concentrations below the normal limit. The unclassified patients showed comparatively rapid LH concentration decreases in all treatment groups as shown in FIG. 9(d) and Table 14(d).

**Table 14(d): Concentrations for LH (mlU/mL) on Each of the Observation Days for Patients Having Unknown-Related Hypogonadism (Summary of Mean ± SD)**

| | **N** | **5 g/day** | **N** | **10 g/day** | **N** | **T-patch** |
|---|---|---|---|---|---|---|
| Day 0 | 17 | 1.8 ± 1.2 | 26 | 2.5 ± 1.5 | 21 | 2.5 ± 1.5 |
| Day 30 | 17 | 0.3 ± 0.3 | 26 | 0.3 ± 0.3 | 21 | 1.3 ± 1.3 |
| Day 60 | 17 | 0.4 ± 0.5 | 26 | 0.3 ± 0.3 | 18 | 1.2 ± 1.4 |
| Day 90 | 17 | 0.5 ± 0.5 | 26 | 0.3 ± 0.4 | 18 | 1.0 ± 1.4 |
| Day 120 | 17 | 0.4 ± 0.4 | 26 | 0.4 ± 0.5 | 12 | 1.2 ± 1.1 |
| Day 150 | 17 | 0.8 ± 1.1 | 23 | 0.3 ± 0.4 | 13 | 1.1 ± 1.1 |
| Day 180 | 15 | 0.3 ± 0.4 | 25 | 0.4 ± 0.4 | 11 | 1.5 ± 1.3 |

### (3) Summary: LH and FSH

Patients receiving AndroGel^{®} or the testosterone patch achieve "hormonal steady state" only after long-term treatment. Specifically, data involving FSH and LH show that these hormones do not achieve steady-state until many weeks after treatment. Because testosterone concentrations are negatively inhibited by FSH and LH testosterone levels do not achieve true steady state until these other hormones also achieve steady state. However, because these hormones regulate only endogenous testosterone (which is small to begin with in hypogonadal men) in an intact feedback mechanism (which may not be present depending on the cause of hypogonadism), the level of FSH and/or LH may have little effect on the actual testosterone levels achieved. The net result is that the patients do not achieve a "hormonal steady state" for testosterone even though the C_{avg}, Cₘᵢₙ, and Cₘₐₓ for testosterone remains relatively constant after a few days of treatment.

### 2. Libido and Sexual Performance

Libido and sexual function were assessed by questionnaires the patients answered daily for seven consecutive days before clinic visits on day 0 and on days 30, 60, 90, 120, 150, and 180 days during gel and patch application. The subjects recorded whether they had sexual day dreams, anticipation of sex, flirting, sexual interaction (e.g., sexual motivation parameters) and orgasm, erection, masturbation, ejaculation, intercourse (*e*.*g*., sexual performance parameters) on each of the seven days. The value was recorded as 0 (none) or 1 (any) for analyses and the number of days the subjects noted a parameter was summed for the seven-day period. The average of the four sexual motivation parameters was taken as the sexual motivation mean score and that of the five sexual performance parameters as the sexual performance mean score (0 to 7).

The subjects also assessed their level of sexual desire, sexual enjoyment, and satisfaction of erection using a seven-point Likert-type scale (0 to 7) and the percent of full erection from 0 to 100%. The subjects rated their mood using a 0 to 7 score. Weekly average scores were calculated. The details of this questionnaire had been described previously. *See* Wang et al., Testosterone Replacement Therapy Improves Mood in Hypogonadal Men - A Clinical Research Center Study, 81 J. CLINICAL ENDOCRINOLOGY & METABOLISM 3578-3583 (1996).

### a. Libido

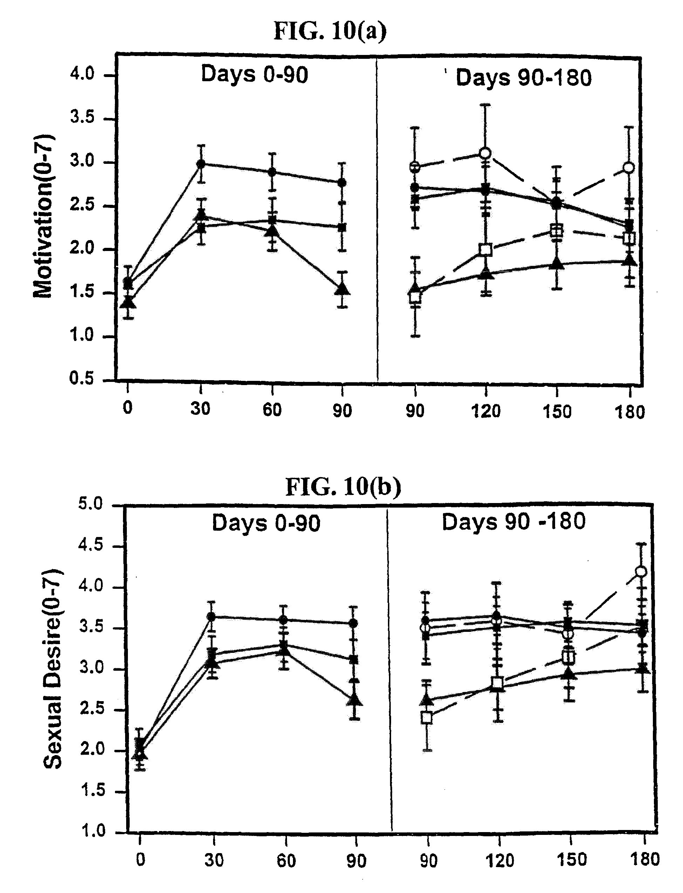

As shown in FIG. 10(a), at baseline, sexual motivation was the same in all treatment groups. After transdermal testosterone treatment, overall sexual motivation showed significant improvement. The change in the summary score from baseline, however, was not different among the three treatment groups.

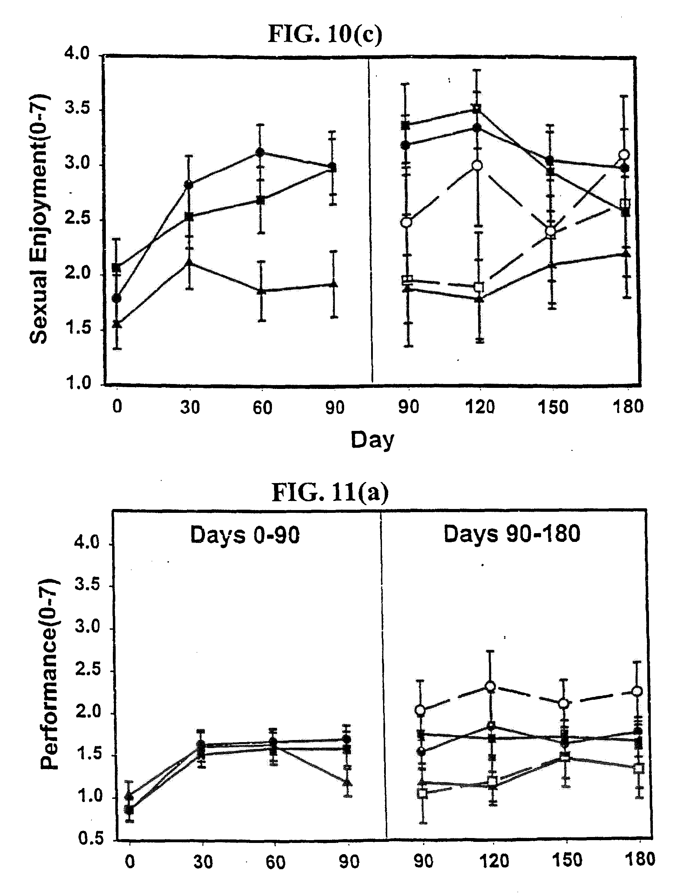

Libido was also assessed from responses on a linear scale of: (1) overall sexual desire, (2) enjoyment of sexual activity without a partner, and (3) enjoyment of sexual activity with a partner. As shown in FIG. 10(b) and Table 15, as a group, overall sexual desire increased after transdermal testosterone treatment without inter-group difference. Sexual enjoyment with and without a partner (FIG. 10(c) and Tables 16 and 17) also increased as a group.

**Table 15: Overall Sexual Desire Changes From Day 0 to Day 180 by Initial Treatment Group (Mean ± SD)**

| **Initial Treatment Group** | **N** | **Day 0** | **N** | **Day 180** | **N** | **Change From Day 0 to Day 180** | **Within-Group p-value** |
|---|---|---|---|---|---|---|---|
| 5.0 g/day T-gel | 69 | 2.1 ± 1.6 | 63 | 3.5 ± 1.6 | 60 | 1.4 ± 1.9 | 0.0001 |
| 10.0 g/ay T-gel | 77 | 2.0 ± 1.4 | 68 | 3.6 ± 1.6 | 67 | 1.5 ± 1.9 | 0.0001 |
| T-Patch | 72 | 2.0 ± 1.6 | 47 | 3.1 ± 1.9 | 45 | 1.6 ± 2.1 | 0.0001 |
| Across-Groups p-value | | 0.8955 | | 0.2247 | | 0.8579 | |

**Table 16: Level of Sexual Enjoyment Without a Partner Changes From Day 0 to Day 180 by Initial Treatment Group (Mean ± SD)**

| **Initial Treatment Group** | **N** | **Day 0** | **N** | **Day 180** | **N** | **Change From Day 0 to Day 180** | **Within-Group p-value** |
|---|---|---|---|---|---|---|---|
| 5.0 g/day T-gel | 60 | 1.5 ± 1.9 | 51 | 1.9 ± 1.9 | 44 | 0.8 ± 1.4 | 0.0051 |
| 10.0 g/day T-gel | 63 | 1.2 ± 1.4 | 53 | 2.2 ± 1.9 | 48 | 1.1 ± 1.6 | 0.0001 |
| T-Patch | 66 | 1.4 ± 1.8 | 44 | 2.2 ± 2.3 | 40 | 1.0 ± 1.9 | 0.0026 |
| Across-Groups p-value | | 0.6506 | | 0.7461 | | 0.6126 | |

**Table 17: Level of Sexual Enjoyment With a Partner Change from Day 0 to Day 180 by Initial Treatment Group (Mean ± SD)**

| **Initial Treatment Group** | **N** | **Day 0** | **N** | **Day 180** | **N** | **Change From Day 0 to Day 180** | **Within-Group p-value** |
|---|---|---|---|---|---|---|---|
| 5.0 g/day T-gel | 64 | 2.1 ± 2.1 | 55 | 2.6 ± 2.2 | 48 | 0.4 ± 2.2 | 0.0148 |
| 10.0 g/day T-gel | 66 | 1.8 ± 1.7 | 58 | 3.0 ± 2.2 | 52 | 1.0 ± 2.3 | 0.0053 |
| T-Patch | 61 | 1.5 ± 1.7 | 40 | 2.2 ± 2.4 | 35 | 0.7 ± 2.3 | 0.1170 |
| Across-Groups p-value | | 0.2914 | | 0.1738 | | 0.3911 | |

### b. Sexual Performance

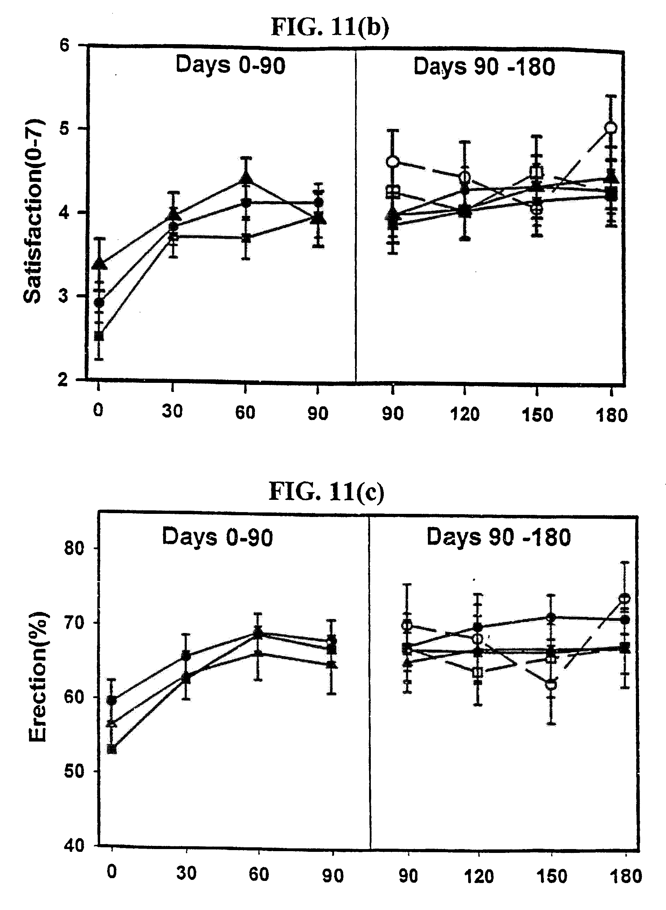

FIG. 11(a) shows that while all treatment groups had the same baseline sexual performance rating, the rating improved with transdermal testosterone treatment in all groups. In addition, as a group, the subjects' self-assessment of satisfaction of erection (FIG. 11(b) and Table 18) and percent full erection (FIG. 11(c) and Table 19) were also increased with testosterone replacement without significant differences between groups. The improvement in sexual function was not related to the dose or the delivery method of testosterone. Nor was the improvement related to the serum testosterone levels achieved by the various testosterone preparations. The data suggest that once a threshold (serum testosterone level probably at the low normal range) is achieved, normalization of sexual function occurs. Increasing serum testosterone levels higher to the upper normal range does not further improve sexual motivation or performance.

**Table 18: Satisfaction with Duration of Erection Change from Day 0 to Day 180 by Initial Treatment Group (Mean ± SD)**

| **Initial Treatment Group** | **N** | **Day 0** | **N** | **Day 180** | **N** | **Change From Day 0 to Day 180** | **Within-Group p-value** |
|---|---|---|---|---|---|---|---|
| 5.0 g/day T-gel | 55 | 2.5 ± 2.1 | 57 | 4.3 ± 1.8 | 44 | 1.9 ± 2.0 | 0.0001 |
| 10/0 g/day T-gel | 64 | 2.9 ± 1.9 | 58 | 4.5 ± 1.7 | 53 | 1.5 ± 2.0 | 0.0001 |
| T-Patch | 45 | 3.4 ± 2.1 | 34 | 4.5 ± 2.0 | 20 | 1.3 ± 2.1 | 0.0524 |
| Across-Groups p-value | | 0.1117 | | 0.7093 | | 0.5090 | |

**Table 19: Percentage of Full Erection Change from Day 0 to Day 180 by Initial Treatment Group (Mean ± SD)**

| **Initial Treatment Group** | **N** | **Day 0** | **N** | **Day 180** | **N** | **Change From Day 0 to Day 180** | **Within-Group p-value** |
|---|---|---|---|---|---|---|---|
| 5.0 g/day T-gel | 53 | 53.1 ± 24.1 | 57 | 67.4 ± 22.5 | 43 | 18.7 ± 22.1 | 0.0001 |
| 10.0 g/day T-gel | 62 | 59.6 ± 22.1 | 59 | 72.0 ± 20.2 | 52 | 10.4 ± 23.4 | 0.0001 |
| T-Patch | 47 | 56.5 ± 24.7 | 33 | 66.7 ± 26.7 | 19 | 12.7 ± 20.3 | 0.0064 |
| Across-Groups p-value | | 0.3360 | | 0.4360 | | 0.1947 | |

### Reference Example 2: Increasing Libido in Eugonadal Men

### Having a Diminished Libido

As discussed above, transdermal application of testosterone using AndroGel^{®} to hypogonadal men results in improved libido and sexual performance. Researchers have found that eugonadal men having a diminished libido have a significant increase in sexual interest after receiving testosterone injections. *See* O'Carrol & Bancroft, Testosterone Therapy for Low Sexual Interest and Erectile Dysfunction in Men: A Controlled Study, Brit. J. Psychiatry 145:146-151 (1984). Thus, the present example describes a method of treating a diminished libido in eugonadal men by transdermal application of a hydroalcoholic testosterone gel to such men. In one embodiment, AndroGel® is applied to the body in accordance with the protocol summarized in Example 1. Libido is measured as in Example 1. Men receiving AndroGel are expected to show an increase in their libido.

### Reference Example 3: Increasing Libido in Eugonadal Men Having a Normal Libido

As discussed above, transdermal application of testosterone using AndroGel^{®} to hypogonadal men results in improved libido and sexual performance. Studies have shown that supra-physiological doses of testosterone administered to eugonadal men having a normal libido resulted in a significant increase in libido. *See* Anderson et al., The Effect of Exogenous Testosterone on Sexuality and Mood of Normal Men, J. CLINICAL ENDOCRINOLOGY & METABOLISM 75:1505-1507 (1992); Bagatel et aL, Metabolic & Behavioral Effects of High-Dose, Exogenous Testosterone in Healthy Men, J. CLINICAL METABOLISM & ENDOCRINOLOGY 79:561-567 (1994). Thus, this example is directed to a method of increasing the libido of normal eugonadal men by application of a transdermal hydroalcoholic testosterone gel. In one embodiment, AndroGel® is applied to the body in accordance with the protocol summarized in Example 1. Libido is measured as in Example 1. Men receiving AndroGel are expected to show an increase in their libido.

### Reference Example 4: Improving Sexual Performance in Eugonadal Men Having Erectile Dysfunction

In a prophetic example, 10 eugonadal males age 18 and older having erectile dysfunction will be randomized to receive: (a) 5.0 g/day of AndroGel® (delivering 50 mg/day of testosterone to the skin of which about 10% or 5 mg is absorbed) for 30 days or (b) 10.0 g/day of AndroGel® (delivering 100 mg/day of testosterone to the skin of which about 10% or 10 mg is absorbed) for 30 days ; or (c) nothing. The effectiveness of AndroGel® in improving sexual performance and treating erecile dysfunction will be evaluated using several assessment instruments. The primary measure will be a sexual function questionnaire, the International Index of Erectile Function ("IIEF"). Two of the questions from the IIEF will serve as primary study endpoints; categorical responses shall be elicited to questions about (1) the ability to achieve erections sufficient for sexual intercourse and (2) the maintenance of erections after penetration. The possible categorical responses to these questions will be (0) no attempted intercourse, (1) never or almost never, (2) a few times, (3) sometimes, (4) most times, and (5) almost always or always. Also collected as part of the IIEF will be information about other aspects of sexual function, including information on erectile function, orgasm, desire, satisfaction with intercourse, and overall sexual satisfaction. Sexual function data shall also be recorded by patients in a daily diary. In addition, patients shall be asked a global efficacy question and an optional partner questionnaire will be administered. In addition, the improvement in erectile dysfunction shall be assessed by an objective measurement of hardness and duration of erections (RigiScan^{®}) with AndroGel treatment compared with placebo. Applicant expects that all test parameters will show improvement over the placebo.

### Reference Example 5: Improving Sexual Performance in Eugonadal Men Having Normal Erections

In a prophetic example, 10 eugonadal males age 18 and older having normal erections (i.e. not diagnosed with erectile dysfunction) will be randomized to receive: (a) 5.0 g/day of AndroGel® (delivering 50 mg/day of testosterone to the skin of which about 10% or 5 mg is absorbed) for 30 days or (b) 10.0 g/day of AndroGel® (delivering 100 mg/day of testosterone to the skin of which about 10% or 10 mg is absorbed) for 30 days ; or (c) nothing. The effectiveness of AndroGel® will be evaluated using several assessment instruments as discussed in Example 4. Applicant expects that all test parameters will show an increase in sexual performance over the placebo. Accordingly, Applicant expects that AndroGel® can be applied to normal men in order to increase the sexual performance above their normal baseline.

### Example 6: Treating Men Having Erectile Dysfunction in Conjunction with other Pharmaceuticals

As discussed above, transdermal application of testosterone using AndroGel^{®} to hypogonadal men results in improved libido and sexual performance. This example is directed to the use of AndroGel in combination with pharmaceuticals useful for treating erectile dysfunction. Such pharmaceuticals include any agent that is effective to inhibit the activity of a phosphodiesterase. Suitable phosphodiesterase inhibitors include, inhibitors of the type III phosphodiesterase (cAMP-specific-cGMP inhibitable form), the type IV phospodiesterase (high affinity-high specificity cAMP form) and the type V phosphodiesterase (the cGMP specific form). Additional inhibitors that may be used in conjunction with the present invention are cGMP-specific phosphodiesterase inhibitors other than type V inhibitors.

Examples of type III phospodiesterase inhibitors that may be administered include, bypyridines such as milrinone and amirinone, imidazolones such as piroximone and enoximone, dihydropyridazinones such as imazodan, 5-methyl-imazodan, indolidan and ICI1118233, quinolinone compounds such as cilostamide, cilostazol and vesnarinone, and other molecules such as bemoradan, anergrelide, siguazodan, trequinsin, pimobendan, SKF-94120, SKF-95654, lixazinone and isomazole.

Examples of type IV phosphodiesterase inhibitors suitable herein include, rolipram and rolipram derivatives such as RO20-1724 nitraquazone and nitraquazone derivatives such as CP-77059 and RS-25344-00, xanthine derivatives such as denbufylline and ICI63197, and other compounds such as EMD54622, LAS-31025 and etazolate.

Examples of type V phosphodiesterase inhibitors include, zaprinast, MY5445, dipyridamole, and sildenafil. Other type V phosphodiesterase inhibitors are disclosed in PCT Publication Nos. WO 94/28902 and WO 96/16644. In the preferred embodiment, an inhibitor of phosphodiesterase type 5 ("PDE5"), such as VIAGRA^{®} (sildenafil citrate USP) is used.

The compounds described in PCT Publication No. WO 94/28902 are pyrazolopyrimidinones. Examples of the inhibitor compounds include 5-(2-ethoxy-5-morpholinoacetylphenyl)-1-methyl-3-n-propyl-1,6-dihydro-7H-p yrazolo[4,3-d]pyrimidin-7-one, 5-(5-morpholinoacetyl-2-n-propoxyphenyl)-1-methyl-3-n-propyl-1,6-dihydro-7 -H-pyrazolo[4,3-d]pyrimidin-7-one, 5-[2-ethoxy-5-(4-methyl-1-piperazinylsulfonyl)-phenyl]1-methyl-3-n-propyl- 1,6-dihydro-7H-pyrazolo[4,3-d]pyrimidin-7-one, 5-[2-allyloxy-5-(4-methyl-1-piperazinylsulfonyl)-phenyl]-1-methyl-3-n-propyl-1,6-dihydro-7H-pyrazolo[4,3-d]pyrimidin-7-one, 5-[2-ethoxy-5-[4-(2-propyl)-1-piperazinylsulfonyl)-phenyl]-1-methyl-3-n-propyl-1,6-dihydro-7H-pyrazolo[4,3-d]pyrimidin-7-one, 5-[2-ethoxy-5-[4-(2-hydroxyethyl)-1-piperazinylsulfonyl)phenyl]-1-methyl-3 -n-propyl-1,6-dihydro-7H-pyrazolo[4,3-d]pyrimidin-7-one, 5-[5-[4-(2-hydroxyethyl)-1-piperazinylsulfonyl]-2-n-propoxyphenyl]-1-methy 1-3-n-propyl-1,6-dihydro-7H-pyrazolo[4,3-d]pyrimidin-7-one, 5[2-ethoxy-5-(4-methyl-1-piperazinylcarbonyl)phenyl]-1-methyl-3-n-propyl-1,6-dihydro-7H-pyrazolo[4,3-d]pyrimidin-7-one, and 5-[2-ethoxy-5-(1-methyl-2-imidazolyl)phenyl]-1-methyl-3-n-propyl-1,6-dihydro-7H-pyrazolo[4,3-d]pyrimidin-7-one.

The phosphodiesterase inhibitors described in PCT Publication No. WO 96/16644 include griseolic acid derivatives, 2-phenylpurinone derivatives, phenylpyridone derivatives, fused and condensed pyrimidines, pyrimidopyrimidine derivatives, purine compounds, quinazoline compounds, phenylpyrimidinone derivative, imidazoquinoxalinone derivatives or aza analogues thereof, phenylpyridone derivatives, and others. Specific examples of the phosphodiesterase inhibitors disclosed in WO 96/16644 include 1,3-dimethyl-5-benzylpyrazolo[4,3-d]pyrimidine-7-one, 2-(2-propoxyphenyl)-6-purinone, 6-(2-propoxyphenyl)-1,2-dihydro-2-oxypyridine-3-carboxamide, 2-(2-propoxyphenyl)-pyrido[2,3-d]pyrimid-4(3H)-one, 7-methylthio-4-oxo-2-(2-propoxyphenyl)-3,4-dihydro-pyrimido[4,5-d]pyrimidi ne, 6-hydroxy-2-(2-propoxyphenyl)pyrimidine-4-carboxamide, 1-ethyl-3-methylimidazo[1,5a]quinoxalin-4(5H)-one, 4-phenylmethylamino-6-chloro-2-(1-imidazoloyl)quinazoline, 5-ethyl-8-[3-(N-cyclohexyl-N-methylcarbamoyl)-propyloxy]-4,5-dihydro-4-oxo-pyrido[3,2-e]-pyrrolo[1,2-a]pyrazine, 5'-methyl-3'-(phenylmethyl)-spiro[cyclopentane-1,7'(8'H)-(3'H)-imidazo[2,1b]purin]4'(5'H)-one, 1-[6-chloro-4-(3,4-methylenedioxybenzyl)-aminoquinazolin-2-yl)piperidine-4-carboxylic acid, (6R, 9S)-2-(4-trifluoromethylphenyl)methyl-5-methyl-3,4,5,6a,7,8,9,9a-octahydrocyclopent[4,5]-midazo[2,1-b]-purin-4-one, 1t-butyl-3-phenylmethyl-6-(4-pyridyl)pyrazolo[3,4-d]-pyrimid-4-one, 1-cyclopentyl-3-methyl-6-(4-pyridyl)-4,5-dihydro-1H-pyrazolo[3,4-d]pyrimid-4-one, 2-butyl-1-(2-chlorobenzyl)6-ethoxy-carbonylbenzimidaole, and 2-(4-carboxypiperidino)-4-(3,4-methylenedioxy-benzyl)amino-6-nitroquinazol ine, and 2-phenyl-8-ethoxycycloheptimidazole.

Still other type V phosphodiesterase inhibitors useful in conjunction with the present invention include: IC-351 (ICOS); 4-bromo-5-(pyridylmethylamino)-6-[3-(4-chlorophenyl)propoxy]-3(2H)pyridazi none; 1-[4-[(1,3-benzodioxol-5-ylmethyl)amiono]-6-chloro-2-quinazolinyl]-4-piper idine-carboxylic acid, monosodium salt; (+)-cis-5,6a,7,9,9,9a-hexahydro-2-[4-(trifluoromethyl)-phenymmethyl-5-meth yl-cyclopent-4,5]imidazo[2,1-b]purin-4(3H)one; furazlocillin; cis-2-hexyl-5-methyl-3,4,5,6a,7,8,9,9a-octahydrocyclopent[4,5]imidazo[2,1- b]purin-4-one; 3-acetyl-1-(2-chlorobenzyl)-2-propylindole-6-carboxylate; 4-bromo-5-(3-pyridylmethylamino)-6-(3-(4-chlorophenyl)propoxy)-3-(2H)pyridazinone; 1-methyl-5-(5-morpholinoacetyl-2-n-propoxyphenyl)-3-n-propyl-1,6-dihydro-7 H-pyrazolo(4,3-d)pyrimidin-7-one; 1-[4-[(1,3-benzodioxol-5-ylmethyl)amino]-6-chloro-2-quinazolinyl]-4-piperi dinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); and Sch-51866.

Other phosphodiesterase inhibitors that may be used in the method of this invention include nonspecific phosphodiesterase inhibitors such as theophylline, IBMX, pentoxifylline and papaverine, and direct vasodilators such as hydralazine.

The active agents may be administered, if desired, in the form of salts, esters, amides, prodrugs, derivatives, and the like, provided the salt, ester, amide, prodrug or derivative is suitable pharmacologically, i.e., effective in the present method. Salts, esters, amides, prodrugs and other derivatives of the active agents may be prepared using standard procedures known to those skilled in the art of synthetic organic chemistry and described, for example, by J. March, Advanced Organic Chemistry: Reactions, Mechanisms and Structure, 4th Ed. (New York: Wiley-Interscience, 1992). For example, acid addition salts are prepared from the free base using conventional methodology, and involves reaction with a suitable acid. Generally, the base form of the drug is dissolved in a polar organic solvent such as methanol or ethanol and the acid is added thereto. The resulting salt either precipitates or may be brought out of solution by addition of a less polar solvent. Suitable acids for preparing acid addition salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid, phosphoric acid, and the like. An acid addition salt may be reconverted to the free base by treatment with a suitable base. Particularly preferred acid addition salts of the active agents herein are halide salts, such as may be prepared using hydrochloric or hydrobromic acids. Conversely, preparation of basic salts of acid moieties which may be present on a phosphodiesterase inhibitor molecule are prepared in a similar manner using a pharmaceutically acceptable base such as sodium hydroxide, potassium hydroxide, ammonium hydroxide, calcium hydroxide, trimethylamine, or the like. Particularly preferred basic salts herein are alkali metal salts, e.g., the sodium salt, and copper salts. Preparation of esters involves functionalization of hydroxyl and/or carboxyl groups which may be present within the molecular structure of the drug. The esters are typically acyl-substituted derivatives of free alcohol groups, i.e., moieties which are derived from carboxylic acids of the formula RCOOH where R is alkyl, and preferably is lower alkyl. Esters can be reconverted to the free acids, if desired, by using conventional hydrogenolysis or hydrolysis procedures. Amides and prodrugs may also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine. Prodrugs are typically prepared by covalent attachment of a moiety, which results in a compound that is therapeutically inactive until modified by an individual's metabolic system.

Other compounds useful for treating erectile dysfunciton may also be used. These include: (a) pentoxifylline (TRENTAL^{®}); (b) yohimbine hydrocholoride (ACTIBINE^{®}, YOCON^{®}, YOHIMEX^{®}); (c) apomorphine (UPRIMA^{®}); (d) alprostadil (the MUSE^{®} system, TOPIGLAN^{®}, CAVERJECT^{®}); (e) papavaerine (PAVABID^{®}, CERESPAN^{®}); (f) phentolamine (VASOMAX^{®}, REGITINE^{®}), and combinations, salts, derivatives and enantiomers of all of the above.

A testosterone containing gel, such as AndroGel® is administered to increase and enhance the therapeutic effectiveness of such drugs, in either hypogonadal or eugonadal men having erectile dysfunction. While pharmaceuticals such as VIAGRA® work principally by various physiological mechanisms of erection initiation and maintenance, the testosterone gel used in accordance with the present invention plays a beneficial role physiologically, and stimulates both sexual motivation (i.e., libido) and sexual performance. Testosterone controls the expression of the nitric oxide synthase gene. *See* Reilly et al., Androgenic Regulation of NO Availability in Rat Penile Erection, 18 J. ANDROLOGY 110 (1997); Park et al., Effects of Androgens on the Expression of Nitric Oxide Synthase mRNAs in Rat Corpous Cavernosum, 83 BJU INT'L. 327 (1999). Thus, testosterone and other androgens clearly play a role in erectile dysfunction. *See* Lugg et al., The Role of Nitric Oxide in Erectile Function, 16 J. ANDROLOGY 2 (1995); Penson et aL, Androgen and Pituitary Control of Penile Nitric Oxide Synthase and Erectile Function In the Rat, 55 BIOLOGY OF REPRODUCTION 576 (1996); Traish et al., Effects of Castration and Androgen Replacement on Erectile Function in a Rabbit Model, 140 ENDOCRINOLOGY 1861 (1999). Moreover, testosterone replacement restores nitric oxide activity. *See* Baba et al. Delayed Testosterone Replacement Restores Nitric Oxide Synthase Containing Nerve Fibres and the Erectile Response in Rat Penis, BJU INT'L 953 (2000); Garban et al., Restoration of Normal Adult Penile Erectile Response in Aged Rats by Long-Term Treatment with Androgens, 53 BIOLOGY OF REPRODUCTION 1365 (1995); Marin et al., Androgen-dependent Nitric Oxide Release in Rat Penis Correlates with Levels of Constitutive Nitric Oxide Synthase Isoenzymes, 61 BIOLOGY OF REPRODUCTION 1012 (1999).

As disclosed herein, adequate blood levels of testosterone are important to erection. In one embodiment, AndroGel® is applied to the body in accordance with the protocol summarized in Example 1. The pharmaceutical(s) for erectile dysfunction is taken in accordance with the prescription requirements. For example, VIAGRA® is generally taken 20-40 minutes before sexual intercourse in 50 mg doses. This combination of therapy is particularly useful in hypogonadal men who need increased testosterone levels in order to optimize the effects of VIAGRA® and the sexual experience as a whole. In essence, a synergistic effect is obtained. AndroGel® is preferably applied to the body for a sufficient number of days so that the steady-state levels of testosterone are achieved.

In a prophetic example, 10 males age 18 and older will be randomized to receive: (a) 5.0 g/day of AndroGel® (delivering 50 mg/day of testosterone to the skin of which about 10% or 5 mg is absorbed) for 30 days plus 50 mg of sildenafil citrate 1 hour before intercourse after at least 1 day of AndroGel® therapy; or (b) 10.0 g/day of AndroGel® (delivering 100 mg/day of testosterone to the skin of which about 10% or 10 mg is absorbed) for 30 days plus 50 mg of sildenafil citrate 1 hour before intercourse after at least 1 day of AndtoGel® therapy; or (c) 5.0 g/day of AndroGel® (delivering 50 mg/day of testosterone) for 30 days and nothing before intercourse. Libido, erections and sexual performance will be studied as in the previous Examples. Applicant expects that all test parameters will show improvement and synergy with the combination.

In another embodiment, the combination therapy comes in the form of kits containing both the testosterone gel and pharmaceutical for erectile dysfunction in amounts sufficient for the proper dosing of the drugs. The kits also contain a set of instructions for the patient.

## Claims

1. Use of a hydroalcoholic gel comprising a steroid in the testosterone synthetic pathway, one or more C1-C4 alcohols, a penetration enhancing agent, a thickener and water, for the manufacture of a medicament for improving the efficacy of a pharmaceutical useful for treating erectile dysfunction in a male subject, wherein the medicament is percutaneously administered to a subject, wherein the pharmaceutical is a phosphodiesterase type 5 inhibitor.

2. The use of claim 1 wherein the steroid in the testosterone synthetic pathway is selected from the group consisting of testosterone, androstenedione, androstenediol, dehydroepiandrosterone, prenenolone and dihydrotestosterone.

3. The use of claim 1 wherein the steroid in the testosterone synthetic pathway is testosterone.

4. The use of any one of claims 1 to 3, wherein the alcohols are selected from the group consisting of ethanol and isopropanol.

5. The use of any one of claims 1 to 3, wherein the alcohol is ethanol.

6. The use of any one of claims 1 to 5, wherein the penetration enhancer comprises at least one of a C8-C22 fatty acid.

7. The use of any one of claims 1 to 5, wherein the penetration enhancing agent is isopropyl myristate.

8. The use of any one of claims 1 to 5, wherein the penetration enhancing agent is isostearic acid.

9. The use of any one of claims 1 to 8, wherein the thickening agent is polyacrylic acid.

10. The use of claim 1, wherein the steroid in the testosterone synthetic pathway is testosterone, the C1-C4 alcohol is ethanol, the penetration enhancing agent is isopropyl myristate and the thickening agent is polyacrylic acid.

11. The use according to claim 10, wherein the hydroalcoholic gel comprises:
(a) 0.1 % to 10% of testosterone;
(b) 0.1% to 5% of isopropyl myristate;
(c) 0.1% to 5% of polyacrylic acid; and
(d) 30% to 98% of ethanol;
wherein the percentages are on a weight to weight basis of the hydroalcoholic gel.

12. The use of any one of claims 1 to 11, wherein the pharmaceutical is sildenafil citrate, a salt or an enantiomer thereof.

13. The use of any one of claims 1 to 12, wherein the subject is hypogonadal.

14. The use of any one of claims 1 to 12, wherein the subject suffers from primary hypogonadism.

15. The use of any one of claims 1 to 12, wherein the subject is eugonadal.

16. The use of any one of claims 1 to 15 wherein the hydroalcoholic gel is administered to the right/left upper arms/shoulders and to the right/left sides of the abdomen once per day on alternate days.

17. A kit for treating erectile dysfunction comprised of a pharmaceutical useful for treating erectile dysfunction in a man and a hydroalcoholic gel as described in any one of claims 1 to 11, wherein the pharmaceutical is a phosphodiesterase type 5 inhibitor.

18. The kit of claim 17, wherein the pharmaceutical is sildenafil citrate, a salt or an enantiomer thereof.

## Patentansprüche

1. Verwendung eines hydroalkoholischen Gels, enthaltend ein Steroid aus dem Testosteronsynthesepfad, einen oder mehrere C1-C4-Alkohole, ein Penetration steigerndes Mittel, ein Verdickungsmittel und Wasser, zur Herstellung eines Medikaments zur Verbesserung der Wirksamkeit eines für die Behandlung von erektilen Dysfunktionen in einem männlichen Patienten brauchbaren Pharmazeutikums, wobei das Medikament dem Patienten perkutan verabreicht wird, und wobei es sich bei dem Pharmazeutikum um einen Inhibitor von Phosphodiesterase Typ 5 handelt.

2. Verwendung nach Anspruch 1, wobei das Steroid aus dem Testosteronsynthesepfad aus der aus Testosteron, Androstendion, Androstendiol, Dehydroepiandrosteron, Prenenolon und Dihydrotestosteron bestehenden Gruppe ausgewählt ist.

3. Verwendung nach Anspruch 1, wobei es sich bei dem Steroid aus dem Testosteronsynthesepfad um Testosteron handelt.

4. Verwendung nach einem der Ansprüche 1 bis 3, wobei die Alkohole aus der aus Ethanol und Isopropanol bestehenden Gruppe ausgewählt sind.

5. Verwendung nach einem der Ansprüche 1 bis 3, wobei es sich bei dem Alkohol um Ethanol handelt.

6. Verwendung nach einem der Ansprüche 1 bis 5, wobei der Penetrationssteigerer wenigstens eine C8-C22-Fettsäure enthält.

7. Verwendung nach einem der Ansprüche 1 bis 5, wobei es sich bei dem Penetration steigernden Mittel um Isopropylmyristat handelt.

8. Verwendung nach einem der Ansprüche 1 bis 5, wobei es sich bei dem Penetration steigernden Mittel um Isostearinsäure handelt.

9. Verwendung nach einem der Ansprüche 1 bis 8, wobei es sich bei dem Verdickungsmittel um Polyacrylsäure handelt.

10. Verwendung nach Anspruch 1, wobei es sich bei dem Steroid aus dem Testosteronsynthesepfad um Testosteron handelt, bei dem C1-C4-Alkohol um Ethanol handelt, bei dem Penetration steigernden Mittel um Isopropylmyristat handelt und bei dem Verdickungsmittel um Polyacrylsäure handelt.

11. Verwendung nach Anspruch 10, wobei das hydroalkoholische Gel:
(a) 0,1 % bis 10% Testosteron;
(b) 0,1 % bis 5% Isopropylmyristat;
(c) 0,1 % bis 5% Polyacrylsäure und
(d) 30% bis 98% Ethanol enthält;
wobei es sich bei den Prozentangaben um Gewichtsprozent vom hydroalkoholischen Gel handelt.

12. Verwendung nach einem der Ansprüche 1 bis 11, wobei es sich bei dem Pharmazeutikum um Sildenafil Citrat, ein Salz oder ein Enantiomer davon handelt.

13. Verwendung nach einem der Ansprüche 1 bis 12, wobei der Patient hypogonadal ist.

14. Verwendung nach einem der Ansprüche 1 bis 12, wobei der Patient an primärem Hypogonadismus leidet.

15. Verwendung nach einem der Ansprüche 1 bis 12, wobei der Patient eugonadal ist.

16. Verwendung nach einem der Ansprüche 1 bis 15, wobei das hydroalkoholische Gel einmal pro Tag täglich abwechselnd auf die rechten/linken Oberarme/Schultern und auf die rechten/linken Seiten des Bauchs aufgetragen wird.

17. Kit zur Behandlung von erektiler Dysfunktion, umfassend ein zur Behandlung von erektiler Dysfunktion in einem Mann brauchbares Pharmazeutikum und ein wie in einem der Ansprüche 1 bis 11 beschriebenes hydroalkoholisches Gel, wobei es sich bei dem Pharmazeutikum um einen Inhibitor von Phosphodiesterase Typ 5 handelt.

18. Kit nach Anspruch 17, wobei es sich bei dem Pharmazeutikum um Sildenafil Citrat, ein Salz oder ein Enantiomer davon handelt.

## Revendications

1. Utilisation d'un gel hydroalcoolique comprenant un stéroïde dans la voie de synthèse de la testostérone, un ou plusieurs alcools en C1-C4, un agent augmentant la pénétration, un épaississant et de l'eau, pour la fabrication d'un médicament pour améliorer l'efficacité d'un produit pharmaceutique utile pour traiter un dysfonctionnement érectile chez un sujet masculin, où le médicament est administré par voie percutanée à un sujet, où le produit pharmaceutique est un inhibiteur de phosphodiestérase de type 5.

2. Utilisation selon la revendication 1 où le stéroïde dans la voie de synthèse de la testostérone est choisi dans le groupe consistant en la testostérone, l'androstènedione, l'androstènediol, la déshydroépiandrostérone, la prénénolone et la dihydrotestostérone.

3. Utilisation selon la revendication 1 où le stéroïde dans la voie de synthèse de la testostérone est la testostérone.

4. Utilisation selon l'une quelconque des revendications 1 à 3 où les alcools sont choisis dans le groupe consistant en l'éthanol et l'isopropanol.

5. Utilisation selon l'une quelconque des revendications 1 à 3 où l'alcool est l'éthanol.

6. Utilisation selon l'une quelconque des revendications 1 à 5 où l'agent augmentant la pénétration comprend au moins un acide gras en C8-C22.

7. Utilisation selon l'une quelconque des revendications 1 à 5 où l'agent augmentant la pénétration est le myristate d'isopropyle.

8. Utilisation selon l'une quelconque des revendications 1 à 5 où l'agent augmentant la pénétration est l'acide isostéarique.

9. Utilisation selon l'une quelconque des revendications 1 à 8 où l'agent épaississant est le poly(acide acrylique).

10. Utilisation selon la revendication 1 où le stéroïde dans la voie de synthèse de la testostérone est la testostérone, l'alcool en C1-C4 est l'éthanol, l'agent augmentant la pénétration est le myristate d'isopropyle et l'agent épaississant est le poly(acide acrylique).

11. Utilisation selon la revendication 10 où le gel hydroalcoolique comprend :
(a) 0,1 % à 10 % de testostérone ;
(b) 0,1 % à 5 % de myristate d'isopropyle ;
(c) 0,1 % à 5 % de poly(acide acrylique) ; et
(d) 30 % à 98 % d'éthanol ;
où les pourcentages sont sur une base du poids au poids du gel hydroalcoolique.

12. Utilisation selon l'une quelconque des revendications 1 à 11 où le produit pharmaceutique est le citrate de sildénafil, un sel ou un énantiomère de celui-ci.

13. Utilisation selon l'une quelconque des revendications 1 à 12 où le sujet est hypogonadique.

14. Utilisation selon l'une quelconque des revendications 1 à 12 où le sujet souffre d'hypogonadisme primaire.

15. Utilisation selon l'une quelconque des revendications 1 à 12 où le sujet est eugonadique.

16. Utilisation selon l'une quelconque des revendications 1 à 15 où le gel hydroalcoolique est administré aux hauts des bras/épaules droit/gauche et aux côtés droit/gauche de l'abdomen une fois par jour un jour sur deux.

17. Kit pour traiter un dysfonctionnement érectile comprenant un produit pharmaceutique utile pour traiter un dysfonctionnement érectile chez un homme et un gel hydroalcoolique tel que décrit dans l'une quelconque des revendications 1 à 11 où le produit pharmaceutique est un inhibiteur de phosphodiestérase de type 5.

18. Kit selon la revendication 17 où le produit pharmaceutique est le citrate de sildénafil, un sel ou un énantiomère de celui-ci.
